# EUROPEAN PATENT APPLICATION

(11) **EP 4 186 522 A1**
(43) Date of publication of application: **31.05.2023**
(21) Application number: 21845945.1
(22) Date of filing: 22.07.2021
(51) Int. Cl.: A61K 39/395, A61K 9/19, A61K 9/08, A61P 35/00, A61P 37/02, C07K 16/28

(54) **PD-L1/LAG-3 BISPECIFIC ANTIBODY PREPARATION, AND PREPARATION METHOD THEREFOR AND USE THEREOF**

(30) Priority: 23.07.2020 CN 202010720248
(71) Applicant: Innovent Biologics (Suzhou) Co., Ltd., Suzhou, Jiangsu 215123 (CN)
(72) Inventor: YAO, Tianyi, Suzhou, Jiangsu 215123 (CN); MA, Yidong, Suzhou, Jiangsu 215123 (CN); WANG, Yinjue, Suzhou, Jiangsu 215123 (CN)
(74) Representative: Sagittarius IP
(86) International application number: PCT/CN2021/107878
(87) International publication number: WO 2022/017468

(57) **Abstract**

The present invention relates to a formulation comprising a PD-L1/LAG-3 bispecific antibody, and particularly to a pharmaceutical formulation comprising a PD-L1/LAG-3 bispecific antibody, a buffer, a stabilizer, a surfactant and a chelating agent. Furthermore, the present invention further relates to therapeutic or prophylactic use of these formulations.

## Description

The present application claims priority to Chinese Patent Application No. 2020107202480 filed on Jul. 23, 2020, the content of which is incorporated herein by reference in its entirety.

### TECHNICAL FIELD

The present invention relates to the field of antibody formulations. Particularly, the present invention relates to a pharmaceutical formulation, more particularly a stable liquid formulation, a lyophilized formulation and a reconstituted stable liquid formulation, comprising a bispecific antibody that binds simultaneously to PD-L1 and LAG-3, a method for preparing the pharmaceutical formulation, and therapeutic and/or prophylactic use of the pharmaceutical formulation.

### BACKGROUND

The stability of a drug is one of the key indexes for ensuring its efficacy and safety. A good formulation is a key prerequisite to keeping the efficacy and safety of a drug over the shelf life. However, due to the complexity of antibodies and their metabolism pathway, it is currently impossible to predict the formulation conditions required to optimize antibody stability. This is particularly essential considering that different antibodies generally have different CDR sequences and antibody structures, and that such sequence and structural differences will result in different stability properties of the antibodies in a solution. Therefore, based on the stringent requirements for safety and efficacy of antibodies for human use, it is necessary to optimize the formulation for each antibody.

LAG-3 is mainly expressed on the cell membrane surface of activated T cells and NK cells and plays a role in inhibiting the activity of immune cells in killing tumor cells. The PD-1/PD-L1 signaling pathway is one of the major mechanisms that have been widely demonstrated clinically to suppress the immune system from clearing tumors. Targeting simultaneously the LAG-3/MHC II signaling pathway and the PD-1/PD-L1 signaling pathway has also been shown to have some synergy in clinical practice. Therefore, the design of a bispecific antibody capable of simultaneously blocking the PD-1/PD-L1 signaling pathway and the LAG-3/MHC II signaling pathway is of great clinical significance.

### BRIEF SUMMARY

The present invention satisfies the above-described need by providing a pharmaceutical formulation comprising a bispecific antibody that specifically binding to PD-L1 and LAG-3. The antibody formulation disclosed herein exhibits excellent stability against a variety of stability-influencing factors.

In one aspect, the present invention therefore provides a liquid antibody formulation comprising: (i) a PD-L1/LAG-3 bispecific antibody, (ii) a buffer, (iii) a stabilizer, and (iv) a surfactant. Preferably, the liquid antibody formulation further comprises (v) a chelating agent.

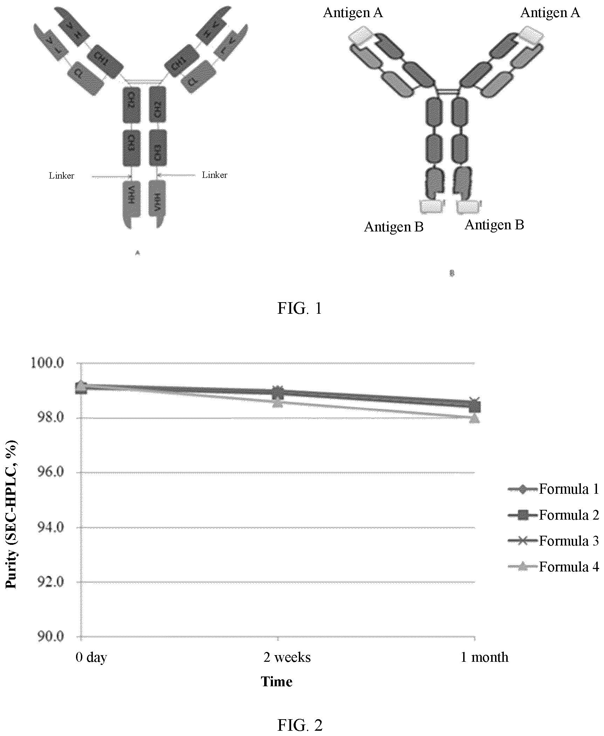

In one embodiment, the structure of the PD-L1/LAG-3 bispecific antibody disclosed herein is shown in FIG. 1, wherein antigen A is LAG-3 and antigen B is PD-L1; the structure comprises:
(i) a polypeptide chain of formula (I):
   VH-CH1-Fc-X-VHH; and
(ii) a polypeptide chain of formula (II):
   VL-CL;
   wherein:
   the VH represents a heavy chain variable region;
   the CH represents a heavy chain constant region;
   the Fc comprises CH2, CH3, and optionally CH4;
   the CH1, the CH2, the CH3 and the CH4 represent domains 1, 2, 3 and 4, respectively, of the heavy chain constant region;
   the X may be absent, or represents a linker when present;
   the VHH represents a single-domain antigen-binding site;
   the VL represents a light chain variable region;
   the CL represents a light chain constant region;
   optionally, a hinge region is present between the CH1 and the Fc.

In one embodiment, the PD-L1/LAG-3 bispecific antibody disclosed herein comprises the sequences shown in the table below:

| Name | Sequence No. |
|---|---|
| VH-CH1-Fc-X-VHH (exemplary polypeptide chain of formula (I)) | SEQ ID NO:1 |
| Anti-LAG-3 antibody VH (exemplary VH of formula (I)) | SEQ ID NO:2 |
| CH1 (exemplary CH1 of formula (I)) | SEQ ID NO:3 |
| Fc (exemplary Fc of formula (I)) | SEQ ID NO:4 |
| Linker (exemplary X of formula (I)) | SEQ ID NO:5 |
| Anti-PD-L1 single-domain antibody (exemplary VHH of formula (I)) | SEQ ID NO:6 |
| VL-CL (exemplary polypeptide chain of formula (II)) | SEQ ID NO:7 |
| Anti-LAG-3 antibody VL (exemplary VL of formula (II)) | SEQ ID NO:8 |
| CL (exemplary CL of formula (II)) | SEQ ID NO:9 |
| CDR1 of anti-PD-L1 single-domain antibody (exemplary VHH CDR1 of formula (I)) | SEQ ID NO:10 |
| CDR2 of anti-PD-L1 single-domain antibody (exemplary VHH CDR2 of formula (I)) | SEQ ID NO:11 |
| CDR3 of anti-PD-L1 single-domain antibody (exemplary VHH CDR3 of formula (I)) | SEQ ID NO:12 |
| Anti-LAG-3 antibody HCDR1 (exemplary HCDR1 of VH of formula (I)) | SEQ ID NO:13 |
| Anti-LAG-3 antibody HCDR2 (exemplary HCDR2 of VH of formula (I)) | SEQ ID NO:14 |
| Anti-LAG-3 antibody HCDR3 (exemplary HCDR3 of VH of formula (I)) | SEQ ID NO:15 |
| Anti-LAG-3 antibody LCDR1 (exemplary LCDR1 of VL of formula (II)) | SEQ ID NO:16 |
| Anti-LAG-3 antibody LCDR2 (exemplary LCDR2 of VL of formula (II)) | SEQ ID NO:17 |
| Anti-LAG-3 antibody LCDR3 (exemplary LCDR3 of VL of formula (II)) | SEQ ID NO:18 |
| Anti-LAG-3 antibody heavy chain (exemplary VH-CH1-Fc of formula (I)) | SEQ ID NO:19 |

| SEQ ID NO | Sequences |
|---|---|
| 1 | QLQLQESGPGLVKPSETLSLTCTVSGGSIYSESYYWGWIRQPPGKGLEWIGSIVY SGYTYYNPSLKSRVTISVDTSKNQFSLKLSSVTAADTAVYYCARVRTWDAAFDI WGQGTMVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHTCPPCPAPEAAGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVM HEALHNHYTQKSLSLSPGGGGGSGGGGSQVQLQESGGGLVQPGGSLRLSCAAS AYTISRNSMGWFRQAPGKGLEGVAAIESDGSTSYSDSVKGRFTISLDNSKNTLYL EMNSLRAEDTAVYYCAAPKVGLGPRTALGHLAFMTLPALNYWGQGTLVTVSS |
| 2 | QLQLQESGPGLVKPSETLSLTCTVSGGSIYSESYYWGWIRQPPGKGLEWIGSIVY SGYTYYNPSLKSRVTISVDTSKNQFSLKLSSVTAADTAVYYCARVRTWDAAFDI WGQGTMVTVSS |
| 3 | ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHT |
| 4 | CPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAP IEKTISKAKGQPREPQVYTLPPSRDELTKNQVSLTCLVKGFYPSDIAVEWESNGQ PENNYKTTPPVLDSDGSFFLYSKLTVDKSRWQQGNVFSCSVMHEALHNHYTQK SLSLSPG |
| 5 | GGGGSGGGGS |
| 6 | QVQLQESGGGLVQPGGSLRLSCAASAYTISRNSMGWFRQAPGKGLEGVAAIESD GSTSYSDSVKGRFTISLDNSKNTLYLEMNSLRAEDTAVYYCAAPKVGLGPRTAL GHLAFMTLPALNYWGQGTLVTVSS |
| 7 | DIQMTQSPSSLSASVGDRVTITCQASQDISNYLNWYQQKPGKAPKLLIYDASNL ETGVPSRFSGSGSGTDFTFTISSLQPEDIATYYCQQVLELPPWTFGGGTKVEIKRT VAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |
| 8 | DIQMTQSPSSLSASVGDRVTITCQASQDISNYLNWYQQKPGKAPKLLIYDASNL ETGVPSRFSGSGSGTDFTFTISSLQPEDIATYYCQQVLELPPWTFGGGTKVEIK |
| 9 | |
| 10 | AYTISRNSMG (AbM rule) |
| 11 | AIESDGSTSYSDSVKG (Kabat rule) |
| 12 | PKVGLGPRTALGHLAFMTLPALNY (Kabat rule) |
| 13 | GSIYSESYYWG |
| 14 | SIVYSGYTYYNPSLKS (Kabat rule) |
| 15 | ARVRTWDAAFDI (IMGT rule) |
| 16 | QASQDISNYLN (Kabat rule) |
| 17 | DASNLET (Kabat rule) |
| 18 | QQVLELPPWT (Kabat rule) |
| 19 | QLQLQESGPGLVKPSETLSLTCTVSGGSIYSESYYWGWIRQPPGKGLEWIGSIVY SGYTYYNPSLKSRVTISVDTSKNQFSLKLSSVTAADTAVYYCARVRTWDAAFDI WGQGTMVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCDKTHTCPPCPAPEAAGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFSCSVM HEALHNHYTQKSLSLSPG |

The PD-L1/LAG-3 bispecific antibody is the bispecific antibody IGN-LP disclosed in PCT application No. PCT/CN2020/073964 (International application date: Jan. 23, 2020).

In one embodiment, the PD-L1/LAG-3 bispecific antibody is recombinantly expressed in HEK 293 cells or CHO cells.

In one embodiment, the PD-L1/LAG-3 bispecific antibody in the liquid antibody formulation disclosed herein is at a concentration of about 10-200 mg/mL. In another embodiment, the PD-L1/LAG-3 bispecific antibody in the liquid antibody formulation disclosed herein is at a concentration of about 20-100 mg/mL, e.g., 20, 25, 30, 35, 40, 45, 50, 55, 60, 70, 80, 90 or 100 mg/mL; preferably, the PD-L1/LAG-3 bispecific antibody is at a concentration of about 20-60 mg/mL, more preferably 20-30 mg/mL.

In one embodiment, the buffer in the liquid antibody formulation disclosed herein is at a concentration of about 5-50 mM. In one embodiment, the buffer in the liquid antibody formulation disclosed herein is at a concentration of about 5-30 mM, e.g., about 5, 10, 15, 20, 25 or 30 mM. In one embodiment, the buffer is a histidine buffer, a citric acid buffer, an acetic acid buffer, or a phosphoric acid buffer; preferably, the buffer is a histidine buffer. In one embodiment, the buffer is a histidine buffer, preferably, the buffer consists of histidine and histidine hydrochloride. In one preferred embodiment, the buffer is a histidine buffer at about 5-30 mM, e.g., about 5-15 mM, e.g., a histidine buffer at about 10 mM. In another embodiment, the histidine buffer used in the formulation disclosed herein consists of, for example, about 0.85 mg/mL histidine and about 0.97 mg/mL histidine hydrochloride.

In one embodiment, the stabilizer in the liquid antibody formulation disclosed herein is selected from polyols (e.g., sorbitol, mannitol or a combination thereof), saccharides (e.g., sucrose, trehalose, maltose or a combination thereof), amino acids (e.g., arginine hydrochloride, methionine, glycine, proline and a combination thereof or a salt thereof) and any combination thereof. In one embodiment, the stabilizer comprises about 10-80 mg/mL sorbitol, e.g., 10, 20, 25, 30, 35, 40, 45, 50, 55, 60, 70 or 80 mg/mL sorbitol, preferably about 15-25 mg/mL sorbitol. In yet another embodiment, the stabilizer comprises about 20-100 mg/mL sucrose, e.g., 20, 30, 40, 50, 60, 70, 80, 90 or 100 mg/mL sucrose, preferably about 40-80 mg/mL sucrose. In one embodiment, the stabilizer comprises arginine, e.g., about 50-200 mM, e.g., 60-180 mM, preferably about 70-170 mM arginine. Preferably, the arginine is arginine hydrochloride.

In one embodiment, the stabilizer comprises arginine as a single component, wherein the arginine is at about 120-200 mM, preferably about 150-180 mM, e.g., 150, 155, 160, 165, 170, 175 or 180 mM, and more preferably about 160-170 mM. Preferably, the arginine is arginine hydrochloride.

In one embodiment, the stabilizer comprises a combination of sorbitol and arginine, wherein the arginine is at about 60-100 mM, preferably about 70-90 mM, e.g., 70, 75, 80, 85 or 90 mM, and the sorbitol is at about 10-30 mg/mL, preferably about 15-25 mg/mL, e.g., 15, 16, 17, 18, 19, 20, 21, 22, 23, 24 or 25 mg/mL. Preferably, the arginine is arginine hydrochloride.

In one embodiment, the stabilizer comprises sucrose as a single component, wherein the sucrose is at about 60-100 mg/mL, preferably about 70-90 mg/mL, e.g. 70, 75, 80, 85 or 90 mg/mL.

In one embodiment, the stabilizer comprises a combination of sucrose and arginine, wherein the arginine is at about 60-100 mM, preferably about 70-90 mM, e.g., 70, 75, 80, 85 or 90 mM, and the sucrose is at about 20-60 mg/mL, preferably about 35-45 mg/mL, e.g., 35, 36, 37, 38, 39, 40, 41, 42, 43, 44 or 45 mg/mL. Preferably, the arginine is arginine hydrochloride.

In one embodiment, the surfactant is a nonionic surfactant. In one embodiment, the surfactant is selected from polysorbate surfactants, poloxamer and polyethylene glycol. In one embodiment, the surfactant is selected from polysorbate surfactants. In one specific embodiment, the surfactant in the liquid antibody formulation disclosed herein is polysorbate 80.

In one embodiment, the surfactant in the liquid antibody formulation disclosed herein is at a concentration of about 0.1-1 mg/mL. In one embodiment, the surfactant in the liquid antibody formulation disclosed herein is at a concentration of about 0.2-0.8 mg/mL, e.g., about 0.2, 0.3, 0.4, 0.5, 0.6, 0.7 or 0.8 mg/mL.

In one embodiment, the chelating agent in the liquid formulation disclosed herein is a carboxylic acid chelating agent. In one embodiment, the chelating agent is selected from edetate disodium, nitrilotriacetic acid, diethylenetriaminepentaacetic acid, citric acid, tartaric acid, gluconic acid, hydroxyethylethylenediaminetriacetic acid, and dihydroxyethylglycine. In one embodiment, the chelating agent is selected from edetate disodium.

In one embodiment, the chelating agent in the liquid antibody formulation disclosed herein is at a concentration of about 0.005-0.05 mg/mL. In one embodiment, the chelating agent in the liquid antibody formulation disclosed herein is at a concentration of about 0.008-0.018 mg/mL, e.g., about 0.008, 0.009, 0.010, 0.012, 0.014 or 0.018 mg/mL.

In one embodiment, the liquid formulation has a pH of about 5.5-6.5. In some embodiments, the liquid formulation has a pH of any value in about 5.5-6.5, e.g., 5.6, 5.8, 6.0, 6.2 or 6.4. Preferably, the formulation has a pH of 5.8-6.4, e.g. a pH of 6.0±0.2 or 6.2±0.2, preferably a pH of 6.0.

In one embodiment, the liquid antibody formulation disclosed herein comprises:
(i) about 20-100 mg/mL, e.g. 20, 25, 30, 35, 40, 45, 50, 55, 60, 70, 80, 90 or 100 mg/mL, PD-L1/LAG-3 bispecific antibody protein, preferably 20-60 mg/mL PD-L1/LAG-3 bispecific antibody protein, and more preferably 20-30 mg/mL PD-L1/LAG-3 bispecific antibody protein;
(ii) about 5-30 mM histidine buffer, preferably about 5-15 mM histidine buffer, and more preferably about 10 mM histidine buffer;
(iii) about 150-180 mM, e.g., 150, 155, 160, 165, 170, 175 or 180 mM, arginine, preferably about 160-170 mM arginine; or a combination of sorbitol and arginine, wherein the arginine is at about 60-100 mM, e.g., 60, 65, 70, 75, 80, 85, 90 or 100 mM, preferably about 70-90 mM, and the sorbitol is at about 10-30 mg/mL, preferably about 15-25 mg/mL, e.g., 15, 16, 17, 18, 19, 20, 21, 22, 23, 24 or 25 mg/mL; or about 60-100 mg/mL sucrose, preferably about 70-90 mg/mL, e.g. 70, 75, 80, 85 or 90 mg/mL, sucrose; or a combination of sucrose and arginine, wherein the arginine is at about 60-100 mM, e.g., 60, 65, 70, 75, 80, 85, 90 or 100 mM, preferably about 70-90 mM, and the sucrose is at about 20-60 mg/mL, preferably about 35-45 mg/mL, such as 35, 36, 37, 38, 39, 40, 41, 42, 43, 44 or 45 mg/mL; and
(iv) about 0.2-0.8 mg/mL, e.g., 0.2, 0.3, 0.4, 0.5, 0.6, 0.7 or 0.8 mg/mL, e.g., 0.3-0.6 mg/mL polysorbate 80; Preferably, the liquid antibody formulation further comprises (v) about 0.008-0.018 mg/mL, for example about 0.008, 0.009, 0.010, 0.012, 0.014 or 0.018 mg/mL edetate disodium;
the liquid formulation has a pH of about 5.5-6.5, preferably a pH of about 5.8-6.4, e.g. a pH of 6.0±0.2 or 6.2±0.2, e.g., about 6.0.

In one preferred embodiment, the liquid antibody formulation comprises:
(i) about 20-100 mg/mL PD-L1/LAG-3 bispecific antibody protein;
(ii) about 5-30 mM histidine buffer;
(iii) about 150-180 mM arginine; or
   a combination of sorbitol and arginine, wherein the arginine is at about 60-100 mM, and the sorbitol is at about 10-30 mg/mL; or
   about 60-100 mg/mL sucrose; or
   a combination of sucrose and arginine, wherein the arginine is at about 60-100 mM, and the sucrose is at about 20-60 mg/mL;
(iv) about 0.2-0.8 mg/mL polysorbate 80; and
(v) about 0.008-0.018 mg/mL edetate disodium;
the liquid formulation has a pH of about 5.8-6.4.

In one preferred embodiment, the liquid antibody formulation comprises:
(i) about 20-60 mg/mL PD-L1/LAG-3 bispecific antibody protein;
(ii) about 5-15 mM histidine buffer;
(iii) about 160-170 mM arginine; or
   a combination of sorbitol and arginine, wherein the arginine is at about 70-90 mM, and the sorbitol is at about 15-25 mg/mL; or
   about 70-90 mg/mL sucrose; or
   a combination of sucrose and arginine, wherein the arginine is at about 70-90 mM, and the sucrose is at about 35-45 mg/mL;
(iv) about 0.3-0.6 mg/mL polysorbate 80; and
(v) about 0.008-0.018 mg/mL edetate disodium;
the liquid formulation has a pH of about 5.8-6.4.

In one preferred embodiment, the liquid antibody formulation comprises:
(i) about 20 mg/mL PD-L1/LAG-3 bispecific antibody, about 10 mM histidine buffer, about 165 mM arginine hydrochloride, about 0.50 mg/mL polysorbate 80 and about 0.01 mg/mL edetate disodium, at a pH of about 6.0; or
(ii) about 20 mg/mL PD-L1/LAG-3 bispecific antibody, about 10 mM histidine buffer, about 80 mM arginine hydrochloride, about 23.66 mg/mL sorbitol, about 0.50 mg/mL polysorbate 80 and about 0.01 mg/mL edetate disodium, at a pH of about 6.0; or
(iii) about 20 mg/mL PD-L1/LAG-3 bispecific antibody, about 10 mM histidine buffer, about 80.00 mg/mL sucrose, about 0.50 mg/mL polysorbate 80 and about 0.01 mg/mL edetate disodium, at a pH of about 6.0; or
(iv) about 20 mg/mL PD-L1/LAG-3 bispecific antibody, about 10 mM histidine buffer, about 80 mM arginine hydrochloride, about 42.00 mg/mL sucrose, about 0.50 mg/mL polysorbate 80 and about 0.01 mg/mL edetate disodium, at a pH of about 6.0.

The liquid formulation disclosed herein can be stably stored for a long period of time, e.g., at least 24 months or longer. In one embodiment, the liquid formulation disclosed herein can be stable after storage at about -80 °C to about 45 °C, e.g., -80 °C, about -30 °C, about -20 °C, about 0 °C, about 5 °C, about 25 °C, about 35 °C, about 38 °C, about 40 °C, about 42 °C or about 45 °C, for at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer.

In one embodiment, the liquid formulation disclosed herein can be stably stored for at least 24 months. In another embodiment, the liquid formulation disclosed herein is stable at a temperature of at least 40 °C. In yet another embodiment, the liquid formulation disclosed herein remains stable at about 2-8 °C for at least 3 months, preferably at least 12 months, and more preferably at least 24 months. In one embodiment, the liquid formulation disclosed herein remains stable at room temperature or, e.g., about 25 °C, for at least 2 months, preferably at least 3 months, and more preferably at least 6 months. In yet another embodiment, the liquid formulation disclosed herein remains stable at about 40 °C for at least 2 weeks, preferably at least 1 month.

In one embodiment, the stability of the formulation can be indicated by detecting changes in the appearance, visible particles, protein content, turbidity, purity, surfactant content, relative binding activity, and/or charge variants of the formulation. In one embodiment, the stability of the liquid formulation disclosed herein can be determined in a forced high temperature stress test, e.g., after storage at 40±2 °C for at least 1 week, 2 weeks or preferably 1 month, or in an accelerated test, e.g., after storage at 25±2 °C for at least 1 month or 2 months, or in a long-term test, e.g., after storage at 5±3 °C for at least 2 months or 3 months, or in a shaking test (e.g. shaking at room temperature in the dark at 650 r/min for 5 days), and/or in a freezing-thawing test (e.g. 6 freezing-thawing repeats at -30 °C/room temperature). In one embodiment, the stability of the liquid formulation disclosed herein is tested relative to an initial value, for example, an initial value on day 0 of storage, or an initial value prior to a shaking or freezing-thawing test.

In one embodiment, the stability of the liquid formulation disclosed herein is visually inspected after storage, after a shaking test or after a freezing-thawing test, wherein the liquid formulation disclosed herein remains a clear to slightly opalescent, colorless to yellowish liquid free of particles in appearance. In one embodiment, no visible particles exist in the formulation upon visual inspection under a clarity detector. In one embodiment, the stability of the liquid formulation disclosed herein is tested after storage, after a shaking test or after a freezing-thawing test by determining the change in protein content, wherein the change rate in protein content is not more than 20%, preferably not more than 10%, e.g., 7-8%, and more preferably not more than 5%, 2% or 1%, relative to an initial value, as measured, for example, by ultraviolet spectrophotometry (UV). In one embodiment, the stability of the liquid formulation disclosed herein is tested after storage, after a shaking test or after a freezing-thawing test by determining the change in turbidity of the liquid formulation disclosed herein, wherein the change is not more than 0.06, preferably not more than 0.05, and more preferably not more than 0.04 or not more than 0.02, relative to an initial value, as measured, for example, by the OD_{350 nm} method. In one embodiment, the stability of the liquid formulation disclosed herein is tested after storage, after a shaking test or after a freezing-thawing test by determining the change in purity of the liquid formulation disclosed herein, wherein the change in monomer purity (or change in main peak) is not more than 10%, e.g., not more than 5%, 4% or 3%, e.g., not more than 2%, preferably no more than 1%, relative to an initial value, as measured by size exclusion chromatography-high performance liquid chromatography (SEC-HPLC). In one embodiment, the stability of the liquid formulation disclosed herein is tested after storage, after a shaking test or after a freezing-thawing test by determining the change in purity of the formulation disclosed herein, wherein the change in monomer purity (or change in main peak) is reduced by no more than 10%, e.g., no more than 5%, 4%, 3%, 2% or 1%, relative to an initial value, as measured by non-reduced capillary electrophoresis-sodium dodecyl sulfate (CE-SDS). In one embodiment, the stability of the liquid formulation disclosed herein is tested after storage, after a shaking test or after a freezing-thawing test by imaged capillary isoelectric focusing (iCIEF), wherein the sum of changes in charge variants (principal component, acidic component and basic component) of the antibody is not more than 50%, e.g., not more than 40%, 30%, 20%, 10% or 5%, and/or the change in principal component is not more than 20%, 15%, 10%, 8% or 5%, relative to an initial value. In one embodiment, the stability of the liquid formulation disclosed herein is tested after storage, after a shaking test or after a freezing-thawing test by direct ELISA, wherein the relative binding activity of the antibody is 70-130%, e.g. 70%, 80%, 90%, 93%, 95%, 98%, 100%, 103%, 105%, 108%, 110%, 115%, 120%, 125% or 130%, preferably 90-110%, relative to an initial value. In one embodiment, the polysorbate 80 content is determined by high performance liquid chromatography-fluorescence detection (HPLC-FLD method) after storage, or after a shaking test, or after a freezing-thawing test; the polysorbate 80 content should be 0.2-0.8 mg/mL, preferably 0.3-0.7 mg/mL.

In one embodiment, the liquid formulation disclosed herein is stable after storage, e.g., at 25 °C for at least 2 months or at 40±2 °C for 1 month, and preferably has one or more of the following characteristics relative to an initial value on day 0 of storage:
(i) a main peak change less than 1%, and/or a purity greater than 96%, preferably greater than 97% or 98% as measured by SEC-HPLC;
(ii) a main peak change less than 2%, and/or a purity greater than 96%, preferably greater than 97% or 98% as measured by non-reduced CE-SDS;
(iii) a sum of changes in components (principal component, acidic component and basic component) not more than 40% and/or a change in the principal component not more than 20% of the PD-L1/LAG-3 bispecific antibody in the formulation as measured by iCIEF,
   for example, a sum of changes not more than about 40% (e.g., not more than 35%, 30%, 25%, 20%, 15% or 10%) or a change in principal component not more than 20% (e.g., not more than 15%, 12%, 10% or 8%) after storage at 40±2 °C for 1 month, or
   for example, a sum of changes not more than about 20% (e.g., not more than 15%, 14%, 13% or 12%) or a change in principal component not more than about 15% (e.g., not more than 10%, 8%, 7%, 6% or 5%) after storage at 25 °C for 2 months; and
(iv) a relative binding activity of the PD-L1/LAG-3 bispecific antibody in the formulation of 70-130%, for example, 90%, 93%, 95%, 98%, 100%, 103%, 105%, 108%, 110%, 115% or 120%, as measured by ELISA.

In one embodiment, the liquid formulation disclosed herein is stable after storage, e.g., at 5±3 °C for at least 6 months, and preferably has one or more of the following characteristics:
(i) in terms of purity, a main peak should be ≥ 95.0%, as measured by SEC-HPLC;
(ii) in terms of purity, a main peak should be ≥ 90.0%, as measured by non-reduced CE-SDS;
(iii) in terms of charge variants, a principal component should be ≥ 58.0%, as measured by iCIEF;
(iv) a biological activity of the PD-L1/LAG-3 bispecific antibody protein in the formulation should be 70-130%, as determined by luciferase reporter gene cell assay; and
(v) the pH value should be 5.8-6.4.

In one aspect, the liquid formulation disclosed herein is a pharmaceutical formulation, preferably an injection, and more preferably a subcutaneous injection or an intravenous injection. In one embodiment, the liquid formulation is an intravenous infusion.

In another aspect, the present invention provides a solid antibody formulation obtained by solidifying the liquid antibody formulation disclosed herein. The solidification treatment is implemented by, e.g., crystallization, spray drying, or lyophilization. In one preferred embodiment, the solid antibody formulation is, e.g., in the form of a lyophilized powder for injection. The solid antibody formulation can be reconstituted in a suitable vehicle prior to use to give a reconstituted formulation of the present invention. The reconstituted formulation is also a liquid antibody formulation disclosed herein. In one embodiment, the suitable vehicle is selected from water for injection, organic solvents for injection (including but not limited to, oil for injection, ethanol, propylene glycol, and the like), and combinations thereof.

In one aspect, the present invention provides a delivery device comprising the liquid antibody formulation or the solid antibody formulation disclosed herein. In one embodiment, the delivery device disclosed herein is provided in the form of a pre-filled syringe comprising the liquid antibody formulation or the solid antibody formulation disclosed herein, e.g., for use in intravenous, subcutaneous, intradermal or intramuscular injection, or intravenous infusion.

In another aspect, the present invention provides a method for delivering the PD-L1/LAG-3 bispecific antibody protein to a subject, e.g., a mammal, comprising administering the liquid antibody formulation or the solid antibody formulation disclosed herein to the subject, the delivery being implemented, e.g., using a delivery device in the form of a pre-filled syringe.

In another aspect, the present invention provides use of the liquid antibody formulation or the solid antibody formulation disclosed herein in preparing a delivery device or a pre-filled syringe or a medicament for simultaneously targeting the LAG-3 signaling pathway and the PD-L1 signaling pathway to treat or prevent a tumor, wherein the tumor is cancer, including but not limited to gastrointestinal cancer, e.g., colon cancer or colorectal cancer or rectal cancer.

The present invention further provides a method for blocking the LAG-3 signaling pathway and/or the PD-L1 signaling pathway to reduce or eliminate the immunosuppressive effect of LAG-3 and/or PD-L1 in a subject by administering to the subject the liquid antibody formulation or the solid antibody formulation disclosed herein or a delivery device (e.g., a pre-filled syringe) or a medicament comprising the liquid antibody formulation or the solid antibody formulation.

The present invention further provides a method for treating a disease, e.g., the tumor described above, in a subject by administering to the subject the liquid antibody formulation or the solid antibody formulation disclosed herein or a delivery device (e.g., a pre-filled syringe) or a medicament comprising the liquid antibody formulation or the solid antibody formulation.

Other embodiments of the present invention will become apparent by reference to the detailed description hereinafter.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to accurate arrangement and means of the embodiments shown in the drawings.
FIG. 1: the structure of the PD-L1/LAG-3 bispecific antibody disclosed herein
FIG. 2: a graph showing the trend of change in purity (SEC-HPLC method) in a formula screening experiment (40±2 °C)
FIG. 3: a graph showing the trend of change in turbidity (OD_{350 nm} method) in a formula screening experiment (40±2 °C)
FIG. 4: a graph showing the trend of change in charge variant-acidic component (iCIEF method) in a formula screening experiment (40±2 °C)
FIG. 5: a graph showing the trend of change in charge variant-principal component (iCIEF method) in a formula screening experiment (40±2 °C)

### SUMMARY

Before the present invention is described in detail, it should be understood that the present invention is not limited to the particular methods or experimental conditions described herein since the methods and conditions may vary. Further, the terms used herein are for the purpose of describing particular embodiments only and are not intended to be limiting.

### Definitions

Unless defined otherwise, all technical and scientific terms used herein have the same meaning as commonly understood by those of ordinary skill in the art. For the purposes of the present invention, the following terms are defined below.

The term "about" used in combination with a numerical value is intended to encompass the numerical values in a range from a lower limit less than the specified numerical value by 5% to an upper limit greater than the specified numerical value by 5%.

The term "and/or", when used to connect two or more options, should be understood to refer to any one of the options or any two or more of the options.

As used herein, the term "comprise" or "include" is intended to include the described elements, integers or steps, but not to exclude any other elements, integers or steps. As used herein, the term "comprise" or "include", unless indicated otherwise, also encompasses the situation where the entirety consists of the described elements, integers or steps. For example, when referring to an antibody variable region "comprising" a particular sequence, it is also intended to encompass an antibody variable region consisting of the particular sequence.

As used herein, the term "antibody" is used in the broadest sense, and refers to a protein comprising an antigen-binding site and encompasses natural and artificial antibodies with various structures, including but not limited to intact antibodies and antigen-binding fragments of antibodies.

The terms "whole antibody", "full-length antibody", "complete antibody" and "intact antibody" are used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. Each heavy chain constant region consists of 3 domains CH1, CH2 and CH3. Each light chain consists of a light chain variable region (abbreviated herein as VL) and a light chain constant region. Each light chain constant region consists of one domain CL. The VH region and the VL region can be further divided into hypervariable regions (complementarity-determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3 and FR4. The constant regions are not directly involved in binding of antibodies to antigens, but exhibit a variety of effector functions.

The term "antibody formulation" refers to a preparation in a form that allows the biological activity of an antibody as an active ingredient to be exerted effectively, and does not contain other components having unacceptable toxicity to a subject to which the formulation is to be administered. Such antibody formulations are generally sterile. Generally, the antibody formulation comprises a pharmaceutically acceptable excipient. A "pharmaceutically acceptable" excipient is an agent that can be reasonably administered to a mammal subject so that an effective dose of the active ingredient used in the formulation can be delivered to the subject. The concentration of the excipient is adapted to the mode of administration and may, for example, be acceptable for injection.

The term "PD-L1/LAG-3 bispecific antibody formulation", herein also referred to as the "antibody formulation disclosed herein", refers to a preparation comprising a PD-L1/LAG-3 bispecific antibody protein as an active ingredient and a pharmaceutically acceptable excipient. The PD-L1/LAG-3 bispecific antibody protein, as the active ingredient, is suitable for therapeutic or prophylactic administration to a human or non-human animal after the PD-L1/LAG-3 bispecific antibody protein is combined with the pharmaceutically acceptable excipient. The antibody formulation disclosed herein can be prepared, for example, as an aqueous liquid formulation, e.g., in a ready-to-use pre-filled syringe, or as a lyophilized formulation to be reconstituted (i.e., redissolved) by dissolution and/or suspension in a physiologically acceptable solution immediately prior to use. In some embodiments, the PD-L1/LAG-3 bispecific antibody protein formulation is in the form of a liquid formulation.

A "stable" antibody formulation refers to a formulation where the antibody retains an acceptable degree of physical and/or chemical stability after storage in specific conditions, after shaking and/or after repeated freezing-thawing. Although the antibody in the antibody formulation may not maintain 100% of its chemical structure after storage for a specific period of time, shaking or repeated freezing-thawing, the antibody formulation is considered "stable" when about 90%, about 95%, about 96%, about 97%, about 98%, or about 99% of the antibody structure or function is generally maintained. In some specific embodiments, the antibody aggregation or degradation or chemical modification is barely detected in the PD-L1/LAG-3 bispecific antibody protein formulation disclosed herein during manufacture, formulation, transportation and long-term storage, resulting in little or even no loss of biological activity of the PD-L1/LAG-3 bispecific antibody protein and exhibiting high stability. In some embodiments, the PD-L1/LAG-3 bispecific antibody protein formulation disclosed herein substantially retains its physical and chemical stability after storage, shaking and/or repeated freezing-thawing. Preferably, the liquid formulation disclosed herein can be stable at room temperature or at 40 °C for at least 2 weeks, and/or at 25 °C for at least 2 months, and/or at 2-8 °C for at least 6 months.

A variety of analytical techniques are known in the art for determining the stability of proteins, see, e.g., Peptide and Protein Drug Delivery, 247-301, Vincent Lee Ed., Marcel Dekker, Inc., New York, N.Y, Pubs (1991) and Jones, A. Adv. Drug Delivery Rev. 10: 29-90 (1993). Stability can be determined at a selected temperature and for a selected storage time. For example, the storage time can be selected based on the expected shelf life of the formulation. Alternatively, an accelerated stability test can be adopted. In some embodiments, the stability test is performed by conducting various stress tests on the antibody formulation. These tests can represent extreme conditions that a formulated antibody formulation may encounter during manufacture, storage or transportation, and can also represent conditions that may accelerate the instability of the antibody in the antibody formulation during a non-manufacture, -storage or -transportation process. For example, a glass vial can be filled with the formulated PD-L1/LAG-3 bispecific antibody protein formulation so as to examine the antibody for stability under high temperature stress.

The antibody can be considered to "maintain its physical stability" in the formulation if the formulation does not exhibit aggregation, precipitation, turbidity and/or denaturation, or exhibits very little aggregation, precipitation, turbidity, and/or denaturation after a period of storage, after a period of shaking, or after repeated freeze-thawing. Safety issues arise as the aggregation of antibodies in the formulation can potentially lead to an increased immune response in a patient. Accordingly, there is a need to minimize or prevent the aggregation of antibodies in the formulation. Light scattering methods can be used to determine visible aggregates in the formulation. SEC-HPLC can be used to determine soluble aggregates in the formulation. In addition, the stability of the formulation can be indicated by visually inspecting the appearance, color and/or clarity of the formulation, or by detecting the turbidity of the formulation by the OD_{350 nm} method, or by determining the purity of the formulation by the non-reduced CE-SDS method. In one embodiment, the stability of the formulation is measured by determining the percentage of antibody monomer in the formulation after storage at a particular temperature for a particular period of time, after shaking or after repeated freezing-thawing, wherein a higher percentage of antibody monomer in the formulation indicates a higher stability of the formulation.

An "acceptable degree" of physical stability can represent that at least about 90% of PD-L1/LAG-3 bispecific antibody protein monomer is detected in the formulation after storage at a specific temperature for a specific period of time, after shaking or after repeated freezing-thawing. In some embodiments, an acceptable degree of physical stability represents at least about 90%, 91%, 92%, 93%, 94%, 95%, 96%, 97%, 98% or 99% of PD-L1/LAG-3 bispecific antibody protein monomer after storage at a particular temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When the physical stability is assessed, the specific temperature at which the pharmaceutical formulation is stored can be any temperature from about -80 °C to about 45 °C, e.g., about -80 °C, about -30 °C, about -20 °C, about 0 °C, about 4-8 °C, about 5 °C, about 25 °C, about 35 °C, about 37 °C, about 40 °C, about 42 °C, or about 45 °C. For example, a pharmaceutical formulation is considered stable if at least about 90%, 91%, 92%, 93%, 94%, 95%, 96%, 97%, 98% or 99% of PD-L1/LAG-3 bispecific antibody protein monomer is detected after storage at about 40±2 °C for 1 month or 4 weeks. A pharmaceutical formulation is considered stable if at least about 90%, 91%, 92%, 93%, 94%, 95%, 96%, 97%, 98% or 99% of PD-L1/LAG-3 bispecific antibody protein monomer is detected after storage at about 25 °C for 2 months. A pharmaceutical formulation is considered stable if at least about 90%, 91%, 92%, 93%, 94%, 95%, 96%, 97%, 98% or 99% of PD-L1/LAG-3 bispecific antibody protein monomer is detected after storage at about 5 °C for 6 months.

The antibody can be considered to "maintain its chemical stability" in the formulation if the antibody in the formulation does not exhibit significant chemical changes after storage for a period of time, after shaking or after repeated freezing-thawing. Most of the chemical instability results from the formation of covalently modified forms of the antibody (e.g., charge variants of the antibody). Basic variants can be formed, for example, by aspartic acid isomerization, and N- and C-terminal modifications; acidic variants can be produced by deamidation, sialylation and saccharification. Chemical stability can be assessed by detecting and/or quantifying chemically altered forms of the antibody. For example, charge variants of the antibody in the formulation can be detected by cation exchange chromatography (CEX) or imaged capillary isoelectric focusing (iCIEF). In one embodiment, the stability of the formulation is measured by determining the percentage change in charge variants of the antibody in the formulation after storage at a specific temperature for a specific period of time, after shaking or after repeated freezing-thawing, wherein a smaller change indicates a higher stability of the formulation.

An "acceptable degree" of chemical stability can represent a percentage change in charge variants (e.g., principal component, acidic component or basic component) in the formulation not more than 40%, e.g., not more than 30% or 20%, or a sum of percentage change in charge variants (principal component, acidic component and basic component) not more than 60%, e.g., not more than 50% or 30%, or a principal component content of no less than 50%, e.g., no less than 60% or 70%, after storage at a specific temperature for a specific period of time, after shaking or after repeated freezing-thawing. In some embodiments, an acceptable degree of chemical stability can represent a percent change in charge variant-principal component not more than about 50%, 40%, 30%, 20% or 15% or a sum of percent change in charge variants not more than about 60%, 50% or 30%, after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When the chemical stability is assessed, the temperature at which the pharmaceutical formulation is stored can be any temperature from about -80 °C to about 45 °C, e.g., about -80 °C, about -30 °C, about -20 °C, about 0 °C, about 4-8 °C, about 5 °C, about 25 °C, or about 45 °C. For example, the pharmaceutical formulation can be considered stable if the percentage change in charge variant-principal component is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 5 °C for 24 months. The pharmaceutical formulation can also be considered stable if the percentage change in charge variant-principal component is less than about 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 25 °C for 2 months. The pharmaceutical formulation can also be considered stable if the percentage change in charge variant-principal component is less than about 50%, 40%, 30%, 20%, 16%, 15%, 14%, 13%, 12%, 10%, 5% or 4% after storage at 40 °C for 1 month.

The term "lyophilized formulation" refers to a composition obtained or obtainable by a lyophilization process of a liquid formulation. Preferably, it is a solid composition having a water content of less than 5%, preferably less than 3%.

The term "reconstituted formulation" refers to a liquid formulation obtained by dissolving and/or suspending a solid formulation (e.g., a lyophilized formulation) in a physiologically acceptable solution.

The term "room temperature" as used herein refers to a temperature of 15-30 °C, preferably 20-27 °C, and more preferably 25 °C.

"Stress conditions" refer to environments that are chemically and/or physically unfavorable to antibody proteins and may result in unacceptable destabilization of the antibody proteins, e.g., high temperature, shaking and freezing-thawing. "High temperature stress" refers to storing the antibody formulation at room temperature or higher (e.g., 40±2 °C) for a period of time. The stability of the antibody formulation can be determined through a high temperature stress accelerated test.

As used herein, the term "parenteral administration" refers to administrations other than intraintestinal and topical administrations, typically by injection or infusion, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In some embodiments, the stable PD-L1/LAG-3 bispecific antibody protein formulation disclosed herein is administered parenterally to a subject. In one embodiment, the PD-L1/LAG-3 bispecific antibody protein formulation disclosed herein is administered by subcutaneous, intradermal, intramuscular or intravenous injection to a subject.

### I. Antibody Formulation

The present invention provides a stable liquid antibody formulation comprising (i) a PD-L1/LAG-3 bispecific antibody, (ii) a buffer, (iii) a stabilizer, and (iv) a surfactant. The antibody formulation has a pH of about 5.5-6.5. Preferably, the liquid antibody formulation further comprises (v) a chelating agent. In one preferred embodiment, the liquid antibody formulation disclosed herein is in the form of an injection.

### (i) PD-L1/LAG-3 bispecific antibody

The PD-L1/LAG-3 bispecific antibody in the antibody formulation disclosed herein is the bispecific antibody IGN-LP disclosed in PCT application No. PCT/CN2020/073964 (International application date: Jan. 23, 2020). In one embodiment, the PD-L1/LAG-3 bispecific antibody is produced by recombinant expression from CHO cells or HEK293 cells. Preferably, the antibody in the liquid formulation disclosed herein exhibits significant anti-tumor activity. For example, administration of the antibody formulation disclosed herein can produce a significant tumor-inhibiting effect in an MC38-huPD-L1 KI tumor-bearing mouse model.

The amount of antibody or antigen-binding fragment thereof in the antibody formulation disclosed herein can vary with the specific desired characteristics of the formulation, the specific environment, and the specific purpose for which the formulation is used. In some embodiments, the antibody formulation is a liquid formulation, which may comprise about 10-200 mg/mL, preferably about 20-100 mg/mL, e.g., about 20, 25, 30, 35, 40, 45, 50, 55, 60, 70, 80, 90 or 100 mg/mL PD-L1/LAG-3 bispecific antibody; preferably, PD-L1/LAG-3 bispecific antibody is at a concentration of about 20-60 mg/mL, more preferably about 20-30 mg/mL.

### (ii) Buffer

Buffers are reagents that can control the pH of a solution within an acceptable range. In some embodiments, the buffer in the formulation disclosed herein can control the pH of the formulation disclosed herein at about 5.5-6.5.

In some embodiments, the formulation disclosed herein comprises a buffer system selected from: a histidine-histidine hydrochloride buffer system, a citric acid-sodium citrate buffer system, an acetic acid-sodium acetate buffer system and a phosphate buffer system, preferably, a histidine-histidine hydrochloride buffer system.

In some embodiments, the buffer used in the formulation of the present invention is a histidine buffer, particularly a buffer system consisting of histidine and histidine hydrochloride.

### (iii) Stabilizer

Suitable stabilizers for use in the present invention can be selected from saccharides, polyols and amino acids and a combination thereof. Saccharides that may be used as a stabilizer include, but are not limited to, sucrose, trehalose, maltose, and a combination thereof. Polyols that may be used as a stabilizer include, but are not limited to, sorbitol, mannitol, and a combination thereof. Amino acids that may be used as a stabilizer include, but are not limited to, arginine, arginine hydrochloride, methionine, glycine, proline, and a combination thereof.

In one embodiment, the liquid formulation disclosed herein comprises arginine as the stabilizer.

In one embodiment, the liquid formulation disclosed herein comprises a combination of arginine and sorbitol as the stabilizer.

In one embodiment, the liquid formulation disclosed herein comprises sucrose as the stabilizer.

In one embodiment, the liquid formulation disclosed herein comprises a combination of sucrose and arginine as the stabilizer.

In one embodiment, the arginine is arginine hydrochloride.

### (iv) Surfactant

As used herein, the term "surfactant" refers to an organic substance with an amphiphilic structure; that is, the structure is composed of groups with opposite solubility tendencies, typically an oil-soluble hydrocarbon chain and a water-soluble ionic group.

In one embodiment, the surfactant in the liquid formulation disclosed herein is a non-ionic surfactant, e.g., alkyl poly(ethylene oxide). Specific non-ionic surfactants that can be contained in the formulation disclosed herein include, for example, polysorbates such as polysorbate 20, polysorbate 80, polysorbate 60 or polysorbate 40, poloxamer, and the like. In one preferred embodiment, the liquid formulation disclosed herein comprises polysorbate 80 as the surfactant.

In some embodiments, surfactants that can be used in the liquid formulation disclosed herein include, but are not limited to, polysorbate-based surfactants (e.g., polysorbate 80, polysorbate 20), poloxamer and polyethylene glycol.

The amount of surfactant in the antibody formulation disclosed herein can vary with the specific desired characteristics of the formulation, the specific environment, and the specific purpose for which the formulation is used.

### (v) Other excipients

The liquid antibody formulation disclosed herein may or may not comprise other excipients. Such other excipients include, for example, antimicrobials, antistatic agents, antioxidants, chelating agents, gelatin, and the like. These and other known pharmaceutical excipients and/or additives suitable for use in the formulation disclosed herein are well known in the art, for example, as listed in "The Handbook of Pharmaceutical Excipients, 4th edition, edited by Rowe et al., American Pharmaceuticals Association (2003); and Remington: the Science and Practice of Pharmacy, 21st edition, edited by Gennaro, Lippincott Williams & Wilkins (2005)".

As used herein, the term "chelating agent" refers to a compound that is capable of forming a chelate with a central atom, and the stability of the complex is greatly increased due to the formation of the chelate.

In one embodiment, the chelating agent in the liquid formulation disclosed herein is a carboxylic acid chelating agent. In one embodiment, the chelating agent is selected from edetate disodium, nitrilotriacetic acid, diethylenetriaminepentaacetic acid, citric acid, tartaric acid, gluconic acid, hydroxyethylethylenediaminetriacetic acid, and dihydroxyethylglycine. In one embodiment, the chelating agent is selected from edetate disodium.

### II. Preparation of Formulation

The present invention provides a stable formulation comprising a PD-L1/LAG-3 bispecific antibody protein. The PD-L1/LAG-3 bispecific antibody protein used in the formulation disclosed herein can be prepared using techniques known in the art for the production of antibodies. For example, the antibody can be recombinantly prepared. In one preferred embodiment, the antibody disclosed herein is recombinantly prepared in 293 cells or CHO cells.

The use of antibodies as active ingredients in drugs is now very common. Techniques for purifying therapeutic antibodies to pharmaceutical grade are well known in the art. For example, Tugcu et al. (Maximizing productivity of chromatography steps for purification of monoclonal antibodies, Biotechnology and Bioengineering 99 (2008) 599-613) described a monoclonal antibody three-column purification method in which ion exchange chromatography (anionic IEX and/or cationic CEX chromatography) is used after a protein A capture step. Kelley et al. (Weak partitioning chromatography for anion exchange purification of monoclonal antibodies, Biotechnology and Bioengineering 101 (2008) 553-566) described a two-column purification method in which a weak partitioning anion exchange resin is used after protein A affinity chromatography.

Generally, monoclonal antibodies recombinantly produced can be purified using conventional purification methods to provide a drug substance with sufficient reproducibility and proper purity for the formulation of antibody formulations. For example, after the antibody is secreted from the recombinant expression cells into the culture medium, the supernatant of the expression system can be concentrated using a commercially available protein concentration filter, e.g., an Amicon ultrafiltration device. Then the antibody can be purified by methods such as chromatography, dialysis and affinity purification. Protein A is suitable as an affinity ligand for the purification of IgG1, IgG2 and IgG4 antibodies. Other antibody purification methods, such as ion exchange chromatography, can also be used. After the antibody with sufficient purity is obtained, a formulation comprising the antibody can be prepared according to methods known in the art.

For example, the preparation can be performed by the following steps: (1) removing impurities such as cells from fermentation broth by centrifuging and clarifying after the fermentation to give a supernatant; (2) capturing an antibody using affinity chromatography (e.g., a protein A column with specific affinity for IgG1, IgG2 and IgG4 antibodies); (3) inactivating viruses; (4) purifying (usually CEX cation exchange chromatography can be adopted) to remove impurities in a protein; (5) filtering the viruses (to reduce the virus titer by, e.g., more than 4 log10); and (6) ultrafiltering/diafiltering (which can be used to allow the protein to be exchanged into a formulation buffer that is favorable for its stability and concentrated to a suitable concentration for injection). See, e.g., B. Minow, P. Rogge, K. Thompson, BioProcess International, Vol. 10, No. 6, 2012, pp. 48-57.

### III. Analytical Method of Formulation

Biologies stability studies typically include real-time stability studies in actual storage conditions (long-term stability studies), accelerated stability studies and forced condition studies. For the stability studies, the study conditions are explored and optimized according to the purpose and the characteristics of the product; stability study protocols, such as long-term, accelerated and/or forced condition studies and the like, should be established according to various influencing factors (such as temperature, repeated freezing-thawing, vibration and the like). Accelerated and forced condition studies are beneficial to understanding the stability of the product in short-term deviations from storage conditions and in extreme conditions, and provide supporting data for the determination of the shelf life and storage conditions.

During the storage, shaking or repeated freezing-thawing of antibody formulations, antibodies may undergo aggregation, degradation or chemical modification, resulting in antibody heterogeneity (including size heterogeneity and charge heterogeneity), aggregates and fragments, etc., which may affect the quality of the antibody formulations. Accordingly, it is necessary to monitor the stability of antibody formulations.

Various methods are known in the art for testing the stability of antibody formulations. For example, the purity of the antibody formulation can be analyzed and the aggregation level of the antibody can be evaluated by methods such as reduced CE-SDS, non-reduced CE-SDS and SEC-HPLC; charge variants in the antibody formulation can be analyzed by capillary isoelectric focusing electrophoresis (cIEF), imaged capillary isoelectric focusing (iCIEF), ion exchange chromatography (IEX), and the like. In addition, the stability of the formulation can be determined quickly by visually inspecting the appearance of the formulation. The change in turbidity of the formulation can also be detected by the OD_{350 nm} method, which gives information about the amount of soluble and insoluble aggregates. In addition, the change in protein content in the formulation can be detected by ultraviolet spectrophotometry (UV method).

### IV. Use of Formulation

The antibody formulation comprising the PD-L1/LAG-3 bispecific antibody protein disclosed herein is used for preventing or treating diseases, such as autoimmune diseases, inflammatory diseases, infections, tumors, etc. For example, the disease is a tumor (e.g., cancer) or an infection. In some embodiments, the tumor is a tumor immune escape. Preferably, the tumor is, for example, colon cancer or colorectal cancer or rectal cancer.

The present invention further provides use of the formulation disclosed herein in preparing a medicament for delivering an PD-L1/LAG-3 bispecific antibody protein to a mammal. The present invention further provides a method for treating or preventing one or more of the above diseases and disorders with the formulation disclosed herein. Preferably, the mammal is a human.

The antibody formulation disclosed herein can be administered to a subject or a patient in a variety of routes. For example, the administration can be performed by infusion or by using a syringe. Accordingly, in one aspect, the present invention provides a delivery device (e.g., a syringe) comprising the antibody formulation disclosed herein (e.g., a pre-filled syringe). The patient will receive an effective amount of the PD-L1/LAG-3 bispecific antibody protein as the primary active ingredient, i.e., an amount sufficient to treat, ameliorate or prevent the disease or disorder of interest.

The following examples are described to assist in understanding the present invention. The examples are not intended to be and should not be interpreted in any way as limiting the protection scope of the present invention.

### Abbreviations

| Abbreviation | Full name |
|---|---|
| CE-SDS | Sodium dodecyl sulfate capillary electrophoresis |
| ELISA | Enzyme-linked immunosorbent assay |
| FLD | Fluorescence detector |
| HPLC | High performance liquid chromatograph |
| iCIEF | Imaged capillary isoelectric focusing |
| SEC-HPLC | Size exclusion chromatography-high performance liquid chromatography |

### DETAILED DESCRIPTION

In order to develop a simple and easy-to-use injection formulation formula suitable for long-term stable storage of the fully human antibody, the influence of different formulation formulas on the antibody quality was investigated by 40 °C forced and 25 °C accelerated stability tests, and finally, a formulation favorable for the stability of the antibody was selected. And the effectiveness of the formula was demonstrated on a pilot scale by long-term stability studies (5±3 °C). The materials and methods used throughout the study are as follows:
Materials and methods

### 1.1. Materials

| Name | Grade | Manufacturer & brand | Catalog No. | Criteria |
|---|---|---|---|---|
| Histidine | Pharmaceutical grade | Ajinomoto, Shanghai | N/A | *Ch.P* (2015 Edition), *USP* |
| Histidine hydrochloride | Pharmaceutical grade | Ajinomoto, Shanghai | N/A | *Ch.P* (2015 Edition) |
| Sorbitol | Pharmaceutical grade | Roquette, French | H20110265 | EP, BP, NF, USP |
| Edetate disodium | Pharmaceutical grade | Avantor, USA | 8995-01 | EP, BP, JP, USP |
| Sucrose | Pharmaceutical grade | Merck, Germany | 1.00892.9050 | *Ch.P* (2015 Edition), *USP* |
| Arginine hydrochloride | Pharmaceutical grade | Ajinomoto, Shanghai | N/A | *Ch.P* (2015 Edition), *USP* |
| Polysorbate 80 | Pharmaceutical grade | Well, Nanjing | Jiangsu MPA Approval No. F15423203 | *Ch.P* (2015 Edition) |
| Capsule filter H4 | N/A | Sartorius, Germany | 5441307H4-OO | N/A |
| Platinum-cured silicone tubing | N/A | Nalgene, USA | 8600-0080 | N/A |
| 6R vial | N/A | Schott, Suzhou | 1142196 | N/A |
| 20 mm rubber stopper | N/A | West, Singapore | 7002-2354 | N/A |
| 20 mm aluminum-plastic cap | N/A | West, Singapore | 5420-1035 | N/A |

| | | | | |
|---|---|---|---|---|
| Note: N/A denotes not applicable. | | | | |

### 1.2. Instruments and equipment

| Name | Manufacturer & brand | Model No. | No. |
|---|---|---|---|
| Electronic balance | Sartorius, Germany | BSA3202S | PD-A1-186 |
| Electronic balance | Mettler, Switzerland | XPE3003S | PD-A 1-247 |
| Constant temperature and humidity chamber | BINDER, Germany | KBF P 720 | PD-A 1-070 |
| Biochemical incubator | Jinghong, Shanghai | SHP-150 | PD-A1-200 |
| Medical refrigerator | Haier, Qingdao | HYC-360 | PD-A1-166 |
| Medical refrigerator | Haier, Qingdao | HYC-360 | PD-A1-165 |
| Ultra-low temperature freezer | Thermo, USA | 907 | PD-A1-175 |
| Clarity detector | Tianda Tianfa, Tianjin | YB-2 | PD-A1-033 |
| Ultraviolet-visible spectrophotometer | Shimadzu, Japan | UV-1800 | AS-A1-037 |
| pH meter | Mettler, Switzerland | FE20 | PD-A1-161 |
| Multi-channel microspectrophotometer | Thermo, USA | Nanodrop 8000 | PD-A1-052 |
| Benchtop refrigerated centrifuge | Thermo, USA | SL16R | PD-A1-082 |
| Clean bench | Airtech, Suzhou | SW-CJ-2FD | QC-A1-011 |
| Medium-flow manual peristaltic pump | Watson Marlow, UK | 520S/R2 | PD-A1-235 |
| Filling machine | Watson Marlow, Denmark | FP50 | PD-C14-115 |
| Insoluble particle detector | Tianda Tianfa, Tianjin | GWJ-8 | QC-A1-094 |

### 1.3. Items and methodology for formulation stability tests

The test items in the whole studies include: (1) the appearance and the presence of visible particles; (2) the protein content in the formulation determined by the ultraviolet method (UV method); (3) the turbidity of the formulation determined by the OD_{350 nm} method; (4) the purity of the antibody formulation determined by size exclusion chromatography-high performance liquid chromatography (SEC-HPLC) and expressed as the percentage of the main peak area to the sum of all peak areas; (5) the purity of the antibody formulation determined by non-reduced capillary electrophoresis-sodium dodecyl sulfate (non-reduced CE-SDS) and expressed as the percentage of the main peak area to the sum of all peak areas; and (6) charge variants in the antibody formulation determined by iCIEF expressed as the percentage of the principal component, acidic component and basic component; (7) the relative binding activity of the PD-L1/LAG-3 bispecific antibody in the antibody formulation to PD-L1 and LAG-3 antigens measured by direct ELISA, or the biological activity of the PD-L1/LAG-3 bispecific antibody in the antibody formulation against PD-L1 and LAG-3 antigens measured by luciferase reporter gene cell assay; (8) the polysorbate 80 content determined by HPLC-FLD.

### Detection of visible particles

The visible particles in the sample were detected using a clarity detector (model No. YB-2, Tianda Tianfa, Tianjin) and an insoluble particle detector (model No. GWJ-8, Tianda Tianfa, Tianjin) according to the method described in the National Pharmacopoeia Committee, the Pharmacopoeia of the People 's Republic of China (2015 edition, volume IV General Rules 0904 "Test for Visible Particles"), Beijing, China Medical Science Press, 2015.

### Determination of protein content

### The protein content of the sample was determined using an ultraviolet spectrophotometer (model No. UV-1800, Shimadzu, Japan) and a multi-channel microspectrophotometer (model No. Nanodrop 8000, Thermo, USA).

### Determination of turbidity

The turbidity of the sample was determined by measuring the absorbance at 350 nm using an ultraviolet spectrophotometer (model No. UV-1800, Shimadzu, Japan).

### Purity (SEC-HPLC)

The separation was performed on an SEC column using a phosphate buffer (3.12 g of sodium dihydrogen phosphate dihydrate, 8.77 g of sodium chloride and 34.84 g of arginine were dissolved in ultra-pure water, the pH was adjusted to 6.8 by adding hydrochloric acid, and the volume was brought to 1000 mL) as the mobile phase. The chromatographic column protective solution was 0.05% (w/v) NaN3, the sample injection volume was 50 µL, the flow rate was 0.5 mL/min, the collection time was 30 min, the column temperature was 25 °C, and the detection wavelength was 280 nm. Test samples were diluted with a sample diluent (0.85 mg/mL histidine, 0.97 mg/mL histidine hydrochloride, 35.11 mg/mL arginine hydrochloride, 0.01 mg/mL edetate disodium and 0.10 mg/mL polysorbate 80) to prepare 2 mg/mL test sample solutions. The formulation buffer was diluted in the same manner as described above to prepare a blank solution. The blank solution and the test sample solution were separately injected into a liquid chromatograph in an amount of 50 µL for determination.

### Purity (non-reduced CE-SDS)

The determination was conducted by capillary gel electrophoresis. The capillary was an uncoated capillary having an inner diameter of 50 µm, a total length of 30.2 cm and an effective length of 20.2 cm. Before electrophoresis, the capillary column was washed with 0.1 mol/L sodium hydroxide, 0.1 mol/L hydrochloric acid, ultra-pure water, and electrophoresis gel at 70 psi. Test samples were diluted to 0.6 mg/mL with a diluent having a pH of 6.5 (200 µL of pH 6.5 citric acid-phosphate buffer was aspirated into 400 µL of 10% sodium dodecyl sulfate, and the solutions were brought to volume of 1 mL with water). 95 µL of the diluted sample was measured out. 5 µL of β-mercaptoethanol was added to reducing samples, and 5 µL of 250 mmol/L NEM (N-ethylmaleimide) was added to non-reducing samples. The mixtures were well mixed, then heated at 70 °C for 10 min, cooled and then transfered to sample tubes. The sampling voltage was -5 kV, the sampling time was 20 seconds, the separation voltage was -12 kV, and the analysis time was 25 minutes.

### Charge variants (iCIEF)

The determination was conducted by imaged capillary isoelectric focusing (iCIEF). The inner diameter of the capillary was 100 µm, and the total length is 5 cm. A sample was diluted to 1.0 mg/mL with a 3 mol/L urea-0.5% MC (hydroxymethyl cellulose) solution. 70 µL of a 3 mol/L urea-0.5% MC solution, 3 µL of ampholyte (pH 3-10), 0.3 µL of each of pI 5.85 and 9.46 Maker (isoelectric point markers), 5 µL of 5 mol/L NDSB (3-[(2-hydroxyethyl)dimethylamino]propane-1-sulfonate), 2 µL of 0.5 mol/L taurine; the final concentration of the sample was 0.2 mg/mL, the focusing voltage was 3 kV, and the focusing time was 5 minutes. The same procedures were performed using the formulation buffer to prepare a blank solution.

### Relative binding activity (direct ELISA)

### Anti-LAG-3 end's binding activity

SA protein was diluted to 1.0 µg/mL with PBS (phosphate-buffered saline), and a 96-well microplate was coated with the protein at 100 µL/well and incubated at 37 °C for 2 h. After being washed, the plate was blocked with a blocking solution (2% BSA-PBST, 300 µL/well) at 37 °C for 2 h. After the plate was washed, Biotin LAG-3 was diluted to 0.5 µg/mL with 2% BSA-PBST (PBST: phosphate-buffered saline + Tween 20; BSA: bovine serum albumin) and added to the microplate at 100 µL/well. The plate was incubated at 37 °C for 30 min and then washed. Test samples were diluted to 40 µg/mL with 2% BSA-PBST and serially diluted 4-fold to the 12th concentration. The serially diluted test samples were added to the washed microplate at 100 µL/well. Wells to which only the diluent was added were used as negative wells. The plate was incubated in an incubator at 37 °C for 1 h. After the plate was washed, an HRP (horseradish peroxidase)-labeled secondary antibody diluted with 2% BSA-PBST (130,000-fold dilution, 100 µL/well) was added. The reactions were carried out at 37 °C for 30 min. After the plate was washed, 100 µL of TMB was added, and after 15 min of chromogenic reaction, 100 µL of 1 mol/L H₂SO₄ was added to each well to terminate the reactions. The OD value at 450 nm was measured with 620 nm as the reference wavelength. Four-parameter curve fitting was performed using GraphPad Prism software with the concentrations of the serial dilutions of the samples as abscissa and the OD450 nm-OD620 nm values of the serial dilutions of the samples as ordinate, and the ratio of reference sample EC50 to sample EC50 was calculated, reflecting the relative binding activity of the test sample to LAG-3.

### Anti-PD-L1 end's binding activity

SA protein was diluted to 1.0 µg/mL with PBS, and a 96-well microplate was coated with the protein at 100 µL/well and incubated at 37 °C for 2 h. After being washed, the plate was blocked with a blocking solution (2% BSA-PBST, 300 µL/well) at 37 °C for 2 h. The plate was washed. Biotin PD-L1 was diluted to 0.5 µg/mL with 2% BSA-PBST and added to the microplate at 100 µL/well. The plate was incubated at 37 °C for 30 min and then washed. Test samples were diluted to 60 µg/mL with 2% BSA-PBST and serially diluted 4-fold to the 12th concentration. The serially diluted test samples were added to the washed microplate at 100 µL/well. Wells to which only the diluent was added were used as negative wells. The plate was incubated in an incubator at 37 °C for 1 h. After the plate was washed, an HRP-labeled secondary antibody diluted with 2% BSA-PBST (100000-fold dilution, 100 µL/well) was added. The reactions were carried out at 37 °C for 30 min. After the plate was washed, 100 µL of TMB was added, and after 15 min of chromogenic reaction, 100 µL of 1 mol/L H₂SO₄ was added to each well to terminate the reactions. The OD value at 450 nm was measured with 620 nm as the reference wavelength. Four-parameter curve fitting was performed using GraphPad Prism software with the concentrations of the serial dilutions of the samples as abscissa and the OD450 nm-OD620 nm values of the serial dilutions of the samples as ordinate, and the ratio of reference sample EC50 to sample EC50 was calculated, reflecting the relative binding activity of the test sample to PD-L1.

### HPLC-FLD method

The polysorbate 80 content of test samples was determined by HPLC-FLD (fluorescence method). The column was a Knitted Reactor coil column (5 m × 0.5 mm ID) from manufacturer SUPELCO. Mobile phase: 0.15 mol/L sodium chloride, 0.05 mol/L Tris, pH 8.0, 5% acetonitrile, 5.0 µmol/L NPN (N-phenyl-1-naphthylamine), 15 ppm Brij solution (polyoxyethylene(23)lauryl ether solution). Detection conditions: flow rate: 1.5 mL/min; excitation wavelength: 350 nm; emission wavelength: 420 nm; column temperature: 30 °C; elution time: 3 minutes; sample injection volume: 10 µL. After the operation was completed, the polysorbate 80 content of the finished product was calculated using the standard curve method.

### Luciferase reporter gene cell assay

### Anti-LAG-3 end's biological activity

The density of MHCII APC cells was adjusted to 0.4 × 10⁶ cells/mL with MHCII APC cells Assay Buffer (1% FBS (fetal bovine serum, catalog No. 10099-141), 99% DMEM-high sugar medium (catalog No. 11995-040)). HA-peptide (catalog No. PP-1901-13) was diluted to 40 µg/mL and mixed with the cells in a ratio, by volume, of 1:1. The mixture was inoculated to a 96-well cell culture plate at 100 µL/well according to an experimental layout, and the cells were incubated overnight (18-22 h). The density of Jurkat-LAG-3-NFAT-Luc2 (catalog No. CS194812) was adjusted to 3.0 × 10⁶ cells/mL with LAG-3 Effector cells Assay Buffer (1% FBS (fetal bovine serum, catalog No. 10099-141), 99% DMEM-high sugar medium (catalog No. 11995-040)). Samples were diluted to 80.00 µg/mL, then serially diluted 4-fold to afford a total of 10 concentration points (0.3-80,000 ng/mL). The media in the 96-well plate was aspirated, and the prepared samples and Jurkat-LAG-3-NFAT-Luc2 cells were added (each at 40 µL/well). The culture was continued for another 7 h. After the cell culture plate was equilibrated to room temperature, 80 µL of Bio-Glo^{™} Luciferase Assay System chromogenic solution (equilibrated in advance to room temperature) was added to each well and reacted for 10 min. The software provided with the Max i3 microplate reader was used, and a reading program was set according to the set layout to read full-wavelength chemiluminescence values. Four-parameter curve fitting was performed using GraphPad Prism software with the concentrations of the samples as abscissa and the Fold values as ordinate, and the ratio of reference sample EC50 to sample EC50 was calculated, reflecting the biological activity of the test sample's anti-LAG-3 end.

### Anti-PD-L1 end's binding activity

The density of CHOK1-PDL1 cells (catalog No. CS187108) was adjusted to 0.3 × 10⁶ cells/mL with Assay Buffer (10% fetal bovine serum, 90% RPMI1640 medium (catalog No. SH30809.01)), and the cells were inoculated to a 96-well culture plate at 100 µL/well according to an experimental layout and subjected to adherent culture for 16-20 h. The density of Jurkat-NFAT-Luc2/PD1 cells (catalog No. CS187102) was adjusted to 0.6 × 10⁶ cells/mL with Assay Buffer. Samples were diluted to 12.00 µg/mL and then serially diluted 2.6-fold to afford a total of 10 concentration points (2.2-12,000 ng/mL). The media in the 96-well plate was aspirated, and the prepared samples and Jurkat-NFAT-Luc2/PD1 cells were added (each at 40 µL/well). The culture was continued for another 6 h. After the cell culture plate was equilibrated to room temperature, 80 µL of Bio-Glo^{™} Luciferase Assay System chromogenic solution (equilibrated in advance to room temperature) was added to each well and reacted for 10 min. The software provided with the Max i3 microplate reader was used, and a reading program was set according to the set layout to read full-wavelength chemiluminescence values. Four-parameter curve fitting was performed using GraphPad Prism software with the concentrations of the samples as abscissa and the Fold values as ordinate, and the ratio of reference sample EC50 to sample EC50 was calculated, reflecting the biological activity of the test sample's anti-PD-L1 end.

### Example 1. Preparation and Purification of PD-L1/LAG-3 Bispecific Antibody

Bispecific antibody IGN-LP simultaneously binding to PD-L1 and LAG-3 was obtained as described in PCT Application No. PCT/CN2020/073964. PCT Application No. PCT/CN2020/073964 is incorporated herein by reference in its entirety.

Briefly, the antibody was recombinantly expressed in HEK293 cells and purified by filtration, chromatography, viral inactivation, filtration, and other processes.

### Example 2. Formula Screening Test

### 2.1. Procedures

A total of 4 formulas were designed, as detailed in Table 1. Buffers of the formulas were prepared, and the purified bispecific antibody protein of Example 1 disclosed herein was exchanged into each of the formula solutions by ultrafiltration. After buffer exchange, proteins of the formulas were diluted to about 20 mg/mL and polysorbate 80 was added. The solutions were filtered and filled into vials. The vials were stoppered and capped, and were subjected to stability tests. The detection items included appearance, visible particles, protein content (UV method), turbidity (OD_{350 nm} method), polysorbate 80 content (HPLC-FLD method), purity (SEC-HPLC and non-reduced CE-SDS), charge variants (iCIEF) and relative binding activity (direct ELISA).

**Table 1. Formula information**

| No. | Formula information |
|---|---|
| Formula 1 | 10 mM (histidine + histidine hydrochloride), 165 mM arginine hydrochloride, 0.01 mg/mL edetate disodium, 0.50 mg/mL polysorbate 80, pH 6.0 |
| Formula 2 | 10 mM (histidine + histidine hydrochloride), 80 mM arginine hydrochloride, 23.66 mg/mL sorbitol, 0.01 mg/mL edetate disodium, 0.50 mg/mL polysorbate 80, pH 6.0 |
| Formula 3 | 10 mM (histidine + histidine hydrochloride), 80 mM arginine hydrochloride, 42.00 mg/mL sucrose, 0.01 mg/mL edetate disodium, 0.50 mg/mL polysorbate 80, pH 6.0 |
| Formula 4 | 10 mM (histidine + histidine hydrochloride), 80.00 mg/mL sucrose, 0.01 mg/mL edetate disodium, 0.50 mg/mL polysorbate 80, pH 6.0 |

Detail on experimental conditions and the sampling schedule is shown in Table 2.

**Table 2. Experimental conditions and sampling schedule**

| Name of experiment | Formulation | Experimental scheme and sampling time points |
|---|---|---|
| 40 °C stability testing | Formulas 1-4 | The solutions were let stand at 40±2 °C, and samples were collected at day 0, week 1, week 2 and month 1. |
| 25 °C stability testing | Formulas 1-4 | The solutions were let stand at 25±2 °C, and samples were collected at month 1 and month 2. |

### 2.2. Criteria

The formula screening test used the criteria in Table 3 to evaluate quality.

**Table 3. Criteria for quality evaluation**

| Test items | Quality criteria |
|---|---|
| Appearance (visual inspection) | Clear to slightly opalescent, colorless to yellowish liquid, free of particles |
| Visible particles (Test for visible particles) | Conforms to the *Pharmacopoeia of the People's Republic of China* (2015 edition, volume IV) 0904[1] |
| Protein content (UV method) | Change rate ≤ 10% |
| Polysorbate 80 content (HPLC-FLD) | Should be 0.3-0.7 mg/mL |
| Turbidity (OD_{350 nm} method) | Change value < 0.02 |
| Purity (SEC-HPLC) | Change in main peak purity ≤ 1% |
| Purity (non-reduced CE-SDS) | Main peak change ≤ 2% |
| Relative binding activity (direct ELISA) | 70-130% |

### 2.3. Results

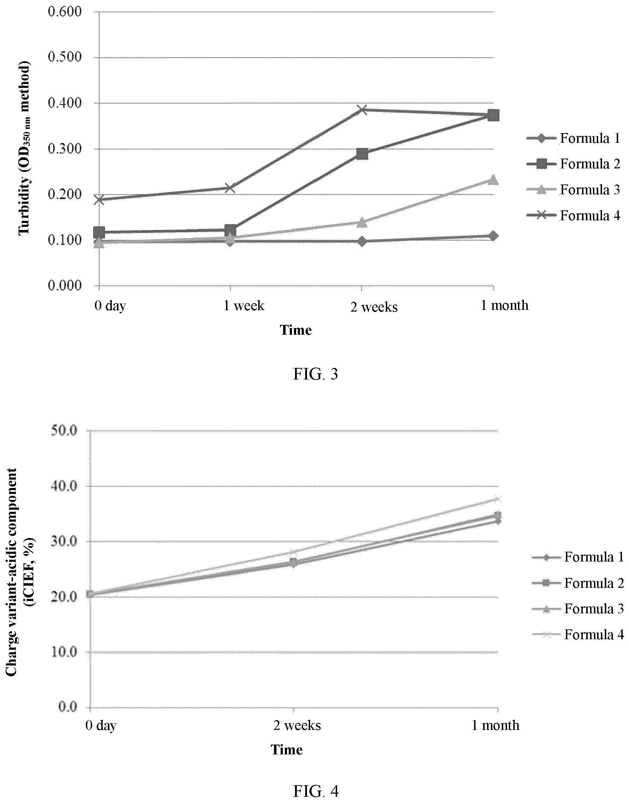

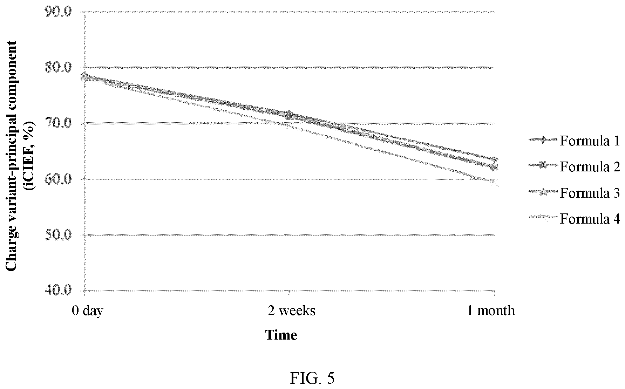

The results of the 40 °C stability testing of the formula screening experiment are detailed in Table 4. The trend of change in purity (SEC-HPLC method) in the formula screening experiment is shown in FIG. 2, the trend of change in turbidity (OD_{350 nm} method) is shown in FIG. 3, and the trend of change in charge variants (iCIEF method) is shown in FIGs. 4-5. After standing at 40±2 °C for 1 month, each formula was acceptable in terms of appearance and visible particles and did not see a significant change in protein content (UV method), polysorbate 80 content (HPLC-FLD method), purity (non-reduced CE-SDS method) and relative binding activity (direct ELISA); formula 4 saw a decrease in purity (SEC-HPLC method), which was reflected by the increased aggregates and fragments; the other formulas did not see a significant change in purity (SEC-HPLC method); each formula saw an increase in turbidity (OD_{350 nm} method), but the increase seen by formula 1 was relatively small; each formula saw an increase in charge variant-acidic component (iCIEF method) and a decrease in principal component, and did not see a significant change in basic component; formula 1 saw a relatively small change in charge variants-acidic component and principal component.

**Table 4. 40 °C stability test results (40±2 °C)**

| Test items | | Time | | Sample name | | | |
|---|---|---|---|---|---|---|---|
| | | | | Formula 1 | Formula 2 | Formula 3 | Formula 4 |
| Appearance (visual inspection) | | 0 day | | Acceptable | Acceptable | Acceptable | Acceptable |
| | | 1 week | | Acceptable | Acceptable | Acceptable | Acceptable |
| | | 2 weeks | | Acceptable | Acceptable | Acceptable | Acceptable |
| | | 1 month | | Acceptable | Acceptable | Acceptable | Acceptable |
| Visible particles (Test for visible particles) | | 0 day | | Acceptable | Acceptable | Acceptable | Acceptable |
| | | 1 week | | Acceptable | Acceptable | Acceptable | Acceptable |
| | | 2 weeks | | Acceptable | Acceptable | Acceptable | Acceptable |
| | | 1 month | | Acceptable | Acceptable | Acceptable | Acceptable |
| Protein content (UV method, mg/mL) | | 0 day | | 22.4 | 21.2 | 21.6 | 20.8 |
| | | 1 month | | 22.0 | 20.5 | 21.3 | 19.6 |
| Turbidity (OD_{350 nm} method) | | 0 day | | 0.096 | 0.117 | 0.095 | 0.189 |
| | | 1 week | | 0.098 | 0.123 | 0.105 | 0.214 |
| | | 2 weeks | | 0.098 | 0.290 | 0.139 | 0.385 |
| | | 1 month | | 0.110 | 0.374 | 0.233 | 0.374 |
| Polysorbate 80 content (HPLC-FLD method) | | 0 day | | 0.5 | 0.5 | 0.5 | 0.6 |
| | | 2 weeks | | 0.5 | 0.5 | 0.5 | 0.5 |
| | | 1 month | | 0.5 | 0.5 | 0.5 | 0.5 |
| Purity (%) | SEC-HPLC | 0day | | 99.2 | 99.1 | 99.1 | 99.2 |
| | | 2weeks | | 99.0 | 98.9 | 99.0 | 98.6 |
| | | 1month | | 98.5 | 98.4 | 98.6 | 98.0 |
| | Non-reduced CE-SDS | 0day | | 98.5 | 98.4 | 98.5 | 98.6 |
| | | 2weeks | | 97.6 | 97.9 | 97.8 | 97.7 |
| | | 1month | | 96.9 | 97.1 | 97.3 | 97.5 |
| Charge variants (iCIEF, %) | Acidic component | 0day | | 20.4 | 20.5 | 20.5 | 20.7 |
| | | 2weeks | | 25.9 | 26.4 | 26.3 | 28.2 |
| | | 1month | | 33.7 | 34.8 | 35.0 | 37.7 |
| | Principal component | 0day | | 78.5 | 78.3 | 78.3 | 78.1 |
| | | 2weeks | | 71.8 | 71.2 | 71.5 | 69.5 |
| | | 1month | | 63.6 | 62.1 | 62.2 | 59.5 |
| | Basic component | 0day | | 1.1 | 1.2 | 1.2 | 1.3 |
| | | 2weeks | | 2.3 | 2.5 | 2.2 | 2.4 |
| | | 1month | | 2.7 | 3.2 | 2.8 | 2.8 |
| Relative binding activity (direct ELISA, %) | | 0 day | Anti-LAG-3e nd | 92 | 86 | 86 | 86 |
| | | | AntiPD-L1en | 89 | 91 | 88 | 82 |

| Formula 1 | Formula 2 | Formula 3 | Formula 4 | | | | |
|---|---|---|---|---|---|---|---|
| | | d | | | | | |
| | 1 month | Anti-LAG-3 end | 87 | 89 | 101 | 87 | |
| | | Anti PD-L1 end | 92 | 88 | 89 | 104 | |

The results of the 25 °C stability testing of the formula screening experiment are detailed in Table 5. After standing at 25±2 °C for 2 months, each formula was acceptable in terms of appearance and visible particles and did not see a significant change in protein content (UV method), turbidity (OD_{350 nm} method), polysorbate 80 content (HPLC-FLD method), purity and relative binding activity (direct ELISA); each formula saw an increase in charge variant-acidic component (iCIEF method) and a decrease in principal component, and did not see a significant change in basic component; formula 4 saw a relatively big change in charge variants-acidic component and principal component.

**Table 5. 25 °C stability test results (25±2 °C)**

| Test items | | Time | Sample name | | | | |
|---|---|---|---|---|---|---|---|
| | | | Formula 1 | Formula 2 | Formula 3 | Formula 4 | |
| Appearance (visual inspection) | | 0 day | Acceptable | Acceptable | Acceptable | Acceptable | |
| | | 1 month | Acceptable | Acceptable | Acceptable | Acceptable | |
| | | 2 months | Acceptable | Acceptable | Acceptable | Acceptable | |
| Visible particles (Test for visible particles) | | 0 day | Acceptable | Acceptable | Acceptable | Acceptable | |
| | | 1 month | Acceptable | Acceptable | Acceptable | Acceptable | |
| | | 2 months | Acceptable | Acceptable | Acceptable | Acceptable | |
| Protein content (UV method, mg/mL) | | 0 day | 22.4 | 21.2 | 21.6 | 20.8 | |
| | | 2 months | 22.5 | 20.2 | 20.4 | 19.4 | |
| Turbidity (OD_{350 nm} method) | | 0 day | 0.096 | 0.117 | 0.095 | 0.189 | |
| | | 1 month | 0.102 | 0.116 | 0.107 | 0.196 | |
| | | 2 months | 0.099 | 0.109 | 0.104 | 0.203 | |
| Polysorbate 80 content (HPLC-FLD method) | | 0 day | 0.5 | 0.5 | 0.5 | 0.6 | |
| | | 1 month | 0.5 | 0.5 | 0.5 | 0.5 | |
| | | 2 months | 0.5 | 0.5 | 0.5 | 0.5 | |
| Purity (%) | SEC-HPLC | 0day | 99.2 | 99.1 | 99.1 | 99.2 | |
| | | 1 month | 99.0 | 99.0 | 99.1 | 99.0 | |
| | | 2months | 98.9 | 98.8 | 98.8 | 98.6 | |
| | Non-reduced CE-SDS | 0day | 98.5 | 98.4 | 98.5 | 98.6 | |
| | | 1 month | 98.4 | 98.4 | 98.5 | 98.4 | |
| | | 2months | 97.6 | 97.7 | 97.6 | 97.6 | |

| Formula 1 | Formula 2 | Formula 3 | Formula 4 | | | | |
|---|---|---|---|---|---|---|---|
| Charge variants (iCIEF, %) | Acidic component | 0 day | | 20.4 | 20.5 | 20.5 | 20.7 |
| | | 1 month | | 22.7 | 23.1 | 23.3 | 23.9 |
| | | 2 months | | 25.6 | 25.8 | 25.9 | 27.4 |
| | Principal component | 0 day | | 78.5 | 78.3 | 78.3 | 78.1 |
| | | 1 month | | 75.5 | 75.1 | 74.7 | 74.1 |
| | | 2 months | | 72.4 | 72.2 | 72.4 | 70.8 |
| | Basic component | 0 day | | 1.1 | 1.2 | 1.2 | 1.3 |
| | | 1 month | | 1.8 | 1.8 | 2.0 | 2.0 |
| | | 2 months | | 2.0 | 2.0 | 1.8 | 1.9 |
| Relative binding activity (direct ELISA, %) | | 0 day | Anti-LAG-3 end | 92 | 86 | 86 | 86 |
| | | | Anti PD-L1 end | 89 | 91 | 88 | 82 |
| | | 2 months | Anti-LAG-3 end | 109 | 87 | 93 | 96 |
| | | | Anti PD-L1 end | 96 | 105 | 102 | 101 |

### Example 3. Long-Term Stability Testing

According to the experimental results of Example 2, formula 1 was selected and subjected to a pilot-test long-term stability test. Three batches of finished formulations were produced on a pilot scale according to formula 1 (20.0 mg/mL recombinant anti-lymphocyte activation gene-3 (LAG-3) and anti-programmed death ligand 1 (PD-L1) bispecific antibody, 0.85 mg/mL histidine, 0.97 mg/mL histidine hydrochloride, 35.11 mg/mL arginine hydrochloride, 0.01 mg/mL edetate disodium, 0.50 mg/mL polysorbate 80, pH 5.8-6.4), subjected to a long-term stability test at 5±3 °C and sampled at months 0, 3 and 6. The results are shown in Table 6. The results show that this formula gives good protein stability, and there were no significant differences between these batches. The formulations meet the quality criteria in the actual production process.

**Table 6. Long-term stability test results (5±3 °C)**

| Test items | Detection method | Quality criteria | Batch No. | 0 month | 3 months | 6 months |
|---|---|---|---|---|---|---|
| Purity | SEC-HPLC | Main peak: should be ≥ 95.0% | 1 | 99.3 | 99.2 | 99.2 |
| | | | 2 | 99.3 | 99.2 | 99.2 |
| | | | 3 | 99.3 | 99.2 | 99.2 |
| | Non-reduced CE-SDS | Main peak: should be ≥ 90.0% | 1 | 97.9 | 97.8 | 97.8 |
| | | | 2 | 97.6 | 97.6 | 97.7 |
| | | | 3 | 97.7 | 97.7 | 97.4 |
| Charge variants | iCIEF | Principal component: should be ≥ 58.0% | 1 | 79.5 | 78.9 | 77.5 |
| | | | 2 | 78.1 | 78.6 | 77.2 |
| | | | 3 | 78.0 | 78.3 | 77.3 |
| | | Acidic component | 1 | 18.9 | 19.5 | 20.7 |
| | | | 2 | 20.3 | 19.9 | 21.1 |
| | | | 3 | 20.4 | 20.0 | 21.1 |
| | | Basic component | 1 | 1.5 | 1.6 | 1.8 |
| | | | 2 | 1.6 | 1.5 | 1.7 |
| | | | 3 | 1.6 | 1.7 | 1.7 |
| Anti-LAG-3 end's biological activity | Luciferase reporter gene cell assay | Should be 70-130% | 1 | 101 | 113 | 105 |
| | | | 2 | 105 | 96 | 89 |
| | | | 3 | 105 | 94 | 111 |
| Biological activity of anti-PD-L1 end | Luciferase reporter gene cell assay | Should be 70-130% | 1 | 101 | 107 | 94 |
| | | | 2 | 99 | 104 | 95 |
| | | | 3 | 100 | 105 | 88 |
| Protein content | UV (gravimetric method) | Should be 18.0-22.0 mg/mL | 1 | 19.7 | 19.6 | 19.7 |
| | | | 2 | 20.3 | 20.3 | 20.4 |
| | | | 3 | 20.6 | 20.8 | 20.8 |
| Appearance | Visual inspection | Should be clear to slightly opalescent, colorless to yellowish liquid, free of particles | 1 | Clear and colorless liquids, free of particles | Slightly opalescent, colorless liquids, free of particles | Clear and colorless liquid, free of particles |
| | | | 2 | Clear and yellowish liquid, free of particles | Slightly opalescent, colorless liquids, free of particles | Clear and colorless liquid, free of particles |
| | | | 3 | Clear and yellowish liquid, free of particles | Slightly opalescent, colorless liquids, free of particles | Clear and colorless liquid, free of particles |
| Visible particles | Test for visible particles | Should comply with specification | 1 | Yes | Yes | Yes |
| | | | 2 | Yes | Yes | Yes |
| | | | 3 | Yes | Yes | Yes |
| pH value | Method for determining pH | Should be 5.8-6.4 | 1 | 6.1 | 6.1 | 6.1 |
| | | | 2 | 6.1 | 6.1 | 6.1 |
| | | | 3 | 6.1 | 6.1 | 6.1 |

### Example 4. Anti-tumor Activity of Anti-LAG-3/PD-Ll Bispecific Antibody Disclosed Herein

The anti-tumor effect of the anti-LAG-3/PD-L1 bispecific antibody IGNLP disclosed herein (i.e., IGN-LP prepared as described in Example 1) was determined using PD-L1 transgenic mice inoculated with MC38-huPD-L1 KI (MJ-1) colon cancer cells (MC38-huPD-L1 KI tumor-bearing mouse models, abbreviated as MC38/PD-L1 models).

MC38-huPD-L1 KI tumor-bearing mouse models were established by subcutaneous inoculation, and the mice were grouped after tumor formation and treated with different antibodies. Tumor volume and body weight change of each group of mice were monitored during administration, wherein the administration frequency was twice a week over 2 weeks, and a total of 5 doses were given. The mice were monitored twice a week over 4 consecutive weeks. Dosages and route of administration are as follows. After the treatment was ended, tumor growth inhibition (TGI%) was calculated by the following formula: TGI% = 100% * (h-IgG control group tumor volume - treatment group tumor volume)/(h-IgG control group tumor volume - h-IgG control group tumor volume before treatment), wherein the average tumor volume of the h-IgG control group was 80 mm³ before treatment.

Mice: PD-L1 transgenic mice, female, 7-8 weeks old (ages at tumor cell inoculation), weighing 18-20 g, purchased from Shanghai Model Organisms Center, Inc. The study started after the mice were acclimated for 7 days after arrival.

Cells: human PD-L1 gene (Nanjing Galaxy Biopharma Co., Ltd.) was knocked in the mouse colon cancer cells MC38 (purchased from OBiO Technology (Shanghai) Co., Ltd., HYC0116) to obtain MC38 cells containing human PD-L1, which were cultured in RPMI 1640 medium (Gibco, 22400-071), and subjected to conventional subculture strictly according to MC38 culture requirements for subsequent *in vivo* experiments. The cells were collected by centrifugation (400 g/min, 5 min) and resuspended in sterile RPMI 1640 basic medium, with the cell density adjusted to 5 × 10⁶ cells/mL. The PD-L1 transgenic mice were subjected to right dorsal shaving and subcutaneous injection of MC38-huPD-L1 KI cells at 0.2 mL/mouse. The tumor volume of each mouse was measured 5 days after inoculation, and mice with the tumor volume ranging from 76 to 80 mm³ were selected and randomly divided into groups by tumor volume. The anti-tumor activity of the anti-LAG-3/PD-L1 antibody used alone was detected by the following administration regimen.

An anti-PD-L1 antibody (humanized anti-PD-L1 antibody Nb-Fc) was obtained using the method described in patent ZL201710657665.3.

An anti-LAG-3 antibody (ADI-31853) was obtained using the method described in Patent Application WO2019/129137.

Administration: the mice were divided into 11 groups (6 mice per group), which were injected subcutaneously with the following doses of antibodies, respectively:
(1) human IgG (equitech-Bio), 15 mg/kg;
(2) anti-PD-L1 antibody (humanized anti-PD-L1 antibody Nb-Fc), 1.25 mg/kg;
(3) anti-PD-L1 antibody (humanized anti-PD-L1 antibody Nb-Fc), 2.5 mg/kg;
(4) anti-PD-L1 antibody (humanized anti-PD-L1 antibody Nb-Fc), 5 mg/kg;
(5) LAG-3 (ADI-31853), 10 mg/kg;
(6) LAG-3 (ADI-31853), 10 mg/kg + anti-PD-L1 antibody (humanized anti-PD-L1 antibody Nb-Fc), 1.25 mg/kg;
(7) LAG-3 (ADI-31853), 10 mg/kg + anti-PD-L1 antibody (humanized anti-PD-L1 antibody Nb-Fc), 2.5 mg/kg;
(8) LAG-3 (ADI-31853), 10 mg/kg + anti-PD-L1 antibody (humanized anti-PD-L1 antibody Nb-Fc), 5 mg/kg;
(9) anti-LAG-3/PD-L1 antibody (IGNLP), 3 mg/kg;
(10) anti-LAG-3/PD-L1 antibody (IGNLP), 6 mg/kg;
(11) anti-LAG-3/PD-L1 antibody (IGNLP), 12 mg/kg.

Human IgG was a preparation of human IgG obtained from Equitech-Bio.

On days 5, 9, 12, 15 and 18 after tumor cell inoculation, mice in each group were administered with the respective antibodies at the dosages described above.

Analysis: the tumor volume and the body weight were measured twice a week throughout the study, and the mice were euthanized when tumors reached an endpoint (the tumor volume was greater than 3000 mm³), or when the mice had more than 20% of weight loss. The maximum length of major axis (L) and maximum length of minor axis (W) of tumors were measured with a vernier caliper, and tumor volume was calculated using the following formula: V=L × W2/2. The tumor volume over time of the mice in various groups was plotted. Statistical significance was determined using analysis of variance (ANOVA). A P value below 0.05 was considered statistically significant in all analyses.

The experimental results are shown in the table below. It can be seen that the anti-LAG-3/PD-L1 antibody IGNLP disclosed herein can significantly inhibit tumor growth alone compared to IgG control (equitech-Bio) or anti-LAG-3 antibody and anti-PD-L1 antibody alone or in combination.

### Tumor Growth Inhibition on Day 29

| Groups | Tumor volume | Tumor growth inhibition (%) | Number of mice with complete regression of tumor |
|---|---|---|---|
| Human IgG | 2411 | - | 0/6 |
| Nb-Fc, 1.25mg/kg | 829 | 68 | 0/6 |
| Nb-Fc, 2.5mg/kg | 1065 | 58 | 0/6 |
| Nb-Fc, 5mg/kg | 902 | 65 | 0/6 |
| ADI-31853 | 2942 | - | 0/6 |
| ADI-31853+Nb-Fc, 1.25mg/kg | 705 | 73 | 0/6 |
| ADI-31853+Nb-Fc, 2.5mg/kg | 1248 | 50 | 0/6 |
| ADI-31853+Nb-Fc, 5mg/kg | 1198 | 52 | 0/6 |
| IGNLP, 3mg/kg | 151 | 97 | 2/6 |
| IGNLP, 6mg/kg | 367 | 88 | 0/6 |
| IGNLP, 12mg/kg | 500 | 82 | 1/6 |

Although specific embodiments of the present invention have been described above, it will be appreciated by those skilled in the art that these embodiments are merely illustrative and that many changes or modifications can be made to these embodiments without departing from the principle and spirit of the present invention. Therefore, the protection scope of the present invention is defined by the appended claims.

## Claims

1. A liquid antibody formulation, comprising:
(i) a PD-L1/LAG-3 bispecific antibody protein,
(ii) a buffer,
(iii) a stabilizer, and
(iv) a surfactant,
wherein the PD-L1/LAG-3 bispecific antibody protein comprises or consists of:
(a) a polypeptide chain of formula (I):
VH-CH1-Fc-X-VHH; and
(b) a polypeptide chain of formula (II):
VL-CL;
wherein:
the VH represents a heavy chain variable region;
the CH represents a heavy chain constant region;
the Fc comprises CH2, CH3, and optionally CH4;
the CH1, the CH2, the CH3 and the CH4 represent domains 1, 2, 3 and 4, respectively, of the heavy chain constant region;
the X may be absent, or represents a linker when present;
the VHH represents a single-domain antigen-binding site;
the VL represents a light chain variable region;
the CL represents a light chain constant region;
optionally, a hinge region is present between the CH1 and the Fc;
wherein the VHH comprises three complementarity-determining regions (VHH CDRs) contained in SEQ ID NO: 6; and/or the VH comprises 3 complementarity-determining regions (HCDRs) of a heavy chain variable region VH set forth in SEQ ID NO: 2; and/or, the VL comprises 3 complementarity-determining regions (LCDRs) of a light chain variable region VL set forth in SEQ ID NO: 8;
preferably, the liquid antibody formulation further comprises (v) a chelating agent;
preferably, the liquid antibody formulation has a pH of about 5.8-6.4, e.g., a pH of 6.0±0.2 or 6.2±0.2,
preferably a pH of about 6.0.

2. The liquid antibody formulation according to claim 1, wherein the PD-L1/LAG-3 bispecific antibody protein in the liquid antibody formulation is at a concentration of about 10-200 mg/mL, preferably about 20-100 mg/mL, e.g., about 20, 25, 30, 35, 40, 45, 50, 55, 60, 70, 80, 90 or 100 mg/mL.

3. The liquid antibody formulation according to claim 1 or 2, wherein
the liquid antibody formulation comprises a buffer system selected from histidine-histidine hydrochloride; preferably, the buffer is at a concentration of about 5-50 mM, preferably about 5-30 mM, e.g., about 5, 10, 15, 20, 25 or 30 mM.

4. The liquid antibody formulation according to any one of claims 1-3, wherein the stabilizer is selected from polyols (e.g., sorbitol, mannitol and a combination thereof), saccharides (e.g., sucrose, trehalose, maltose and a combination thereof), amino acids (e.g., arginine, arginine hydrochloride, methionine, glycine, proline and a combination thereof) and any combination thereof,
for example, the stabilizer comprises one or more selected from:
- a polyol selected from sorbitol, mannitol and a combination thereof;
- a saccharide selected from sucrose, trehalose, maltose and a combination thereof;
- an amino acid selected from arginine hydrochloride, methionine, glycine, proline and a combination thereof.

5. The liquid antibody formulation according to any one of claims 1-4, wherein the stabilizer comprises:
(i) about 120-200 mM arginine, preferably about 150-180 mM, e.g., 150, 155, 160, 165, 170, 175 or 180 mM, arginine, or
(ii) a combination of sorbitol and arginine, wherein the arginine is at about 60-100 mM, preferably about 70-90 mM, e.g., 70, 75, 80, 85 or 90 mM, and the sorbitol is at about 10-30 mg/mL, preferably about 15-25 mg/mL, e.g., 15, 16, 17, 18, 19, 20, 21, 22, 23, 24 or 25 mg/mL, or
(iii) about 60-100 mg/mL sucrose, preferably about 70-90 mg/mL, e.g., 70, 75, 80, 85 or 90 mg/mL, sucrose, or
(iv) a combination of sucrose and arginine, wherein the arginine is at about 60-100 mM, preferably about 70-90 mM, e.g., 70, 75, 80, 85 or 90 mM, and the sucrose is at about 20-60 mg/mL, preferably about 35-45 mg/mL, e.g., 35, 36, 37, 38, 39, 40, 41, 42, 43, 44 or 45 mg/mL;
preferably, the arginine is arginine hydrochloride.

6. The liquid antibody formulation according to any one of claims 1-5, wherein the surfactant in the liquid antibody formulation is selected from a polysorbate surfactant, poloxamer, polyethylene glycol and a combination thereof; polysorbate 80 is preferred.

7. The liquid antibody formulation according to any one of claims 1-6, wherein the surfactant is at a concentration of about 0.1-1 mg/mL, preferably about 0.2-0.8 mg/mL, e.g., about 0.2, 0.3, 0.4, 0.5, 0.6, 0.7 or 0.8 mg/mL.

8. The liquid antibody formulation according to any one of claims 1-7, wherein the chelating agent is a carboxylic acid chelating agent, preferably edetate disodium.

9. The liquid antibody formulation according to any one of claims 1-8, wherein the chelating agent is at a concentration of about 0.008-0.018 mg/mL, e.g., about 0.008, 0.009, 0.010, 0.012, 0.014 or 0.018 mg/mL.

10. The liquid antibody formulation according to any one of claims 1-9, wherein the VHH comprises complementarity-determining regions (CDRs) VHH CDR1, VHH CDR2 and VHH CDR3, wherein the VHH CDR1 comprises or consists of an amino acid sequence set forth in SEQ ID NO: 10, the VHH CDR2 comprises or consists of an amino acid sequence set forth in SEQ ID NO: 11, and the VHH CDR3 comprises or consists of an amino acid sequence set forth in SEQ ID NO: 12;
and/or the VH comprises complementarity-determining regions (CDRs) HCDR1, HCDR2 and HCDR3, wherein the HCDR1 comprises or consists of an amino acid sequence of SEQ ID NO: 13; the HCDR2 comprises or consists of an amino acid sequence of SEQ ID NO: 14; the HCDR3 comprises or consists of an amino acid sequence of SEQ ID NO: 15;
and/or the VL comprises complementarity-determining regions (CDRs) LCDR1, LCDR2, and LCDR3, wherein the LCDR1 comprises or consists of an amino acid sequence of SEQ ID NO: 16; the LCDR2 comprises or consists of an amino acid sequence of SEQ ID NO: 17; the LCDR3 comprises or consists of an amino acid sequence of SEQ ID NO: 18.

11. The liquid antibody formulation according to any one of claims 1-10, wherein the VHH comprises or consists of a sequence set forth in SEQ ID NO: 6; and/or the VH comprises or consists of an amino acid sequence set forth in SEQ ID NO: 2; and/or, the VL comprises or consists of an amino acid sequence set forth in SEQ ID NO: 8.

12. The liquid antibody formulation according to any one of claims 1-11, wherein the VH-CH1-Fc in formula (I) comprises or consists of an amino acid sequence of SEQ ID NO: 19; and/or the VL-CL in formula (II) comprises or consists of an amino acid sequence of SEQ ID NO: 7;
preferably:
the polypeptide chain of formula (I) comprises or consists of a sequence set forth in SEQ ID NO: 1, and/or
the polypeptide chain of formula (II) comprises or consists of a sequence set forth in SEQ ID NO: 7.

13. The liquid antibody formulation according to any one of claims 1-12, wherein the PD-L1/LAG-3 bispecific antibody is recombinantly expressed in HEK293 cells or CHO cells.

14. The liquid antibody formulation according to any one of claims 1-13, wherein the liquid formulation is an injection, preferably for subcutaneous or intravenous injection, or an infusion, e.g., for intravenous infusion.

15. The liquid antibody formulation according to any one of claims 1-14, wherein the liquid antibody formulation comprises:
(i) about 20-100 mg/mL PD-L1/LAG-3 bispecific antibody protein;
(ii) about 5-30 mM histidine buffer;
(iii) about 150-180 mM arginine; or
a combination of sorbitol and arginine, wherein the arginine is at about 60-100 mM, and the sorbitol is at about 10-30 mg/mL; or
about 60-100 mg/mL sucrose; or
a combination of sucrose and arginine, wherein the arginine is at about 60-100 mM, and the sucrose is at about 20-60 mg/mL;
(iv) about 0.2-0.8 mg/mL polysorbate 80; and
(v) about 0.008-0.018 mg/mL edetate disodium;
the liquid formulation has a pH of about 5.8-6.4;
alternatively, the liquid antibody formulation comprises
(i) about 20-60 mg/mL PD-L1/LAG-3 bispecific antibody protein;
(ii) about 5-15 mM histidine buffer;
(iii) about 160-170 mM arginine; or
a combination of sorbitol and arginine, wherein the arginine is at about 70-90 mM, and the sorbitol is at about 15-25 mg/mL; or
about 70-90 mg/mL sucrose; or
a combination of sucrose and arginine, wherein the arginine is at about 70-90 mM, and the sucrose is at about 35-45 mg/mL;
(iv) about 0.3-0.6 mg/mL polysorbate 80; and
(v) about 0.008-0.018 mg/mL edetate disodium;
the liquid formulation has a pH of about 5.8-6.4;
alternatively, the liquid antibody formulation comprises
(i) about 20 mg/mL PD-L1/LAG-3 bispecific antibody, about 10 mM histidine buffer, about 165 mM arginine hydrochloride, about 0.50 mg/mL polysorbate 80 and about 0.01 mg/mL edetate disodium, at a pH of about 6.0; or
(ii) about 20 mg/mL PD-L1/LAG-3 bispecific antibody, about 10 mM histidine buffer, about 80 mM arginine hydrochloride, about 23.66 mg/mL sorbitol, about 0.50 mg/mL polysorbate 80 and about 0.01 mg/mL edetate disodium, at a pH of about 6.0; or
(iii) about 20 mg/mL PD-L1/LAG-3 bispecific antibody, about 10 mM histidine buffer, about 80.00 mg/mL sucrose, about 0.50 mg/mL polysorbate 80 and about 0.01 mg/mL edetate disodium, at a pH of about 6.0; or
(iv) about 20 mg/mL PD-L1/LAG-3 bispecific antibody, about 10 mM histidine buffer, about 80 mM arginine hydrochloride, about 42.00 mg/mL sucrose, about 0.50 mg/mL polysorbate 80 and about 0.01 mg/mL edetate disodium, at a pH of about 6.0.

16. The liquid antibody formulation according to any one of claims 1-15, wherein the formulation is stable after storage, e.g., at 25 °C for at least 2 months, or at 40±2 °C for 1 month, and preferably has one or more of the following characteristics:
(i) a main peak change less than 1%, and/or a purity greater than 96%, preferably greater than 97% or 98% as measured by SEC-HPLC;
(ii) a main peak change less than 2%, and/or a purity greater than 95%, preferably greater than 96% or 97% as measured by non-reduced CE-SDS;
(iii) a sum of changes in components (principal component, acidic component and basic component) not more than 40% and/or a change in the principal component not more than 20% of the PD-L1/LAG-3 bispecific antibody protein in the formulation relative to an initial value on day 0 of storage as measured by iCIEF, for example, a sum of changes not more than about 40% (e.g., not more than 30%) and/or a change in the principal component not more than about 20% (e.g., not more than 15%) after storage at 40±2 °C for 1 month, or a sum of changes not more than about 20% (e.g., about 15%) or a change in the principal component not more than about 15% (e.g., not more than about 10%) after storage at 25 °C for 2 months; and
(iv) a relative binding activity of the PD-L1/LAG-3 bispecific antibody protein in the formulation of 70-130%, e.g., 90-110%, relative to an initial value on day 0 of storage as measured by ELISA;
alternatively, the formulation is stable after storage at 5±3 °C for at least 6 months, and preferably has one or more of the following characteristics:
(i) in terms of purity, a main peak should be ≥ 95.0%, as measured by SEC-HPLC;
(ii) in terms of purity, a main peak should be ≥ 90.0%, as measured by non-reduced CE-SDS;
(iii) in terms of charge variants, a principal component should be ≥ 58.0%, as measured by iCIEF;
(iv) a relative binding activity of the PD-L1/LAG-3 bispecific antibody protein in the formulation should be 70-130%, as determined by luciferase reporter gene cell assay; and
(v) a pH value should be 5.8-6.4.

17. A solid antibody formulation, obtained by solidifying the liquid antibody formulation according to any one of claims 1-16, wherein the solid antibody formulation is, e.g., in the form of a lyophilized powder for injection.

18. A delivery device, comprising the liquid antibody formulation according to any one of claims 1-16 or the solid antibody formulation according to claim 17.

19. A pre-filled syringe, comprising the liquid antibody formulation according to any one of claims 1-16 or the solid antibody formulation according to claim 17 for use in intravenous or intramuscular injection.

20. Use of the liquid antibody formulation according to any one of claims 1-16 or the solid antibody formulation according to claim 17 in preparing a delivery device, a pre-filled syringe or a medicament that blocks LAG-3 and/or PD-L1 pathways to reduce or eliminate immunosuppressive effect in a subject.

21. Use of the liquid antibody formulation according to any one of claims 1-16 or the solid antibody formulation according to claim 17 in preparing a delivery device, a pre-filled syringe or a medicament for treating or preventing a tumor in a subject, wherein for example, the tumor is gastrointestinal cancer.
